# EUROPEAN PATENT APPLICATION

(11) **EP 4 678 632 A1**
(43) Date of publication of application: **14.01.2026**
(21) Application number: 24767181.1
(22) Date of filing: 06.03.2024
(51) Int. Cl.: C07C 319/14, C07C 321/28, C07C 323/20, C07C 381/00, C07D 209/30, C07D 213/36, C07D 213/70, C07D 215/36, C07D 239/38, C07D 241/44, C07D 263/57, C07D 295/033, C07D 307/64, C07D 307/91, C07D 317/62, C07D 319/18, C07D 473/40, C07B 61/00

(54) **METHOD FOR PRODUCING ARYL COMPOUND CONTAINING TRITYLSULFANYL GROUP**

(30) Priority: 06.03.2023 JP 2023033954; 07.11.2023 JP 2023190006
(71) Applicant: AGC Inc., Chiyoda-ku Tokyo 100-8405 (JP); The University of Tokyo, Bunkyo-ku, Tokyo 113-8654 (JP)
(72) Inventor: OKAZOE, Takashi, Tokyo 100-8405 (JP); NOZAKI, Kyoko, Tokyo 113-8654 (JP); GATZENMEIER, Tim, Tokyo 113-8654 (JP); LIU, Yue, Tokyo 113-8654 (JP)
(74) Representative: TBK
(86) International application number: PCT/JP2024/008539
(87) International publication number: WO 2024/185814

(57) **Abstract**

The present invention provides: a substrate for efficiently synthesizing a tritylsulfanyl group-containing aryl compound; a method for producing an SF₅ group-containing compound using the same; and the like. The present invention is a method for producing a tritylsulfanyl group-containing aryl compound, the method including thiotritylation of a halogenated aryl compound represented by the following general formula (1) [wherein A¹ is an aryl group which may have a substituent or a heteroaryl group which may have a substituent; and X is a halogen atom] using [(triphenylmethyl)sulfanyl]potassium or [(triphenylmethyl)sulfanyl]sodium to produce a tritylsulfanyl group-containing aryl compound represented by the following general formula (2) [wherein A¹ is the same as A¹ in the general formula (1); and Ph is a phenyl group].
[Chemical Formula 1]

A¹-X (1)

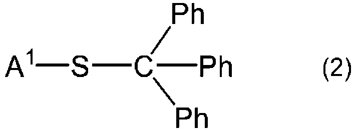

## Description

### TECHNICAL FIELD

The present invention relates to a method for producing a tritylsulfanyl group-containing aryl compound in which a tritylsulfanyl group is introduced into an aryl group.

Priority is claimed on Japanese Patent Application No. 2023-33954, filed March 6, 2023 and Japanese Patent Application No. 2023-190006, filed November 7, 2023, the contents of which are incorporated herein by reference.

### BACKGROUND ART

The pentafluorosulfanyl (SF₅) group has a relatively small size, high electron-withdrawing property, excellent stability against hydrolysis, and improved lipophilicity, and is therefore regarded as a "super trifluoromethyl (CF₃) group" with excellent characteristics. On the other hand, it is difficult to introduce the SF₅ group into an existing compound, and for this reason, despite its great appeal, the use of the SF₅ group in the active ingredients of medicines and agricultural chemicals, organic materials, and the like has not progressed very far.

There are various methods for synthesizing a compound in which SF₅ has been introduced into an aryl group such as a phenyl group. For example, there is a method in which aryl disulfide is directly fluorinated using fluorine gas (F₂) (Patent Document 1), and a method in which aryl disulfide is fluorinated using chlorine gas (Cl₂) and potassium fluoride to obtain aryl tetrafluorosulfanyl chloride (Ar-SF₄Cl), which is then fluorinated with zinc fluoride (ZnF₂) or the like to obtain aryl sulfur pentafluoride (Ar-SF₅) (Patent Document 2). In addition, as a method using silver fluoride (II) (AgF₂), there is a method in which a pentafluorosulfanyl group-containing aryl compound is synthesized from a thioaryl compound in a single step by using silver fluoride (II) and a tetraalkylammonium halide (Patent Document 3).

On the other hand, the tritylsulfanyl group is a group that is widely used as a protecting group for free thiols. A compound in which the tritylsulfanyl group is introduced into an aryl or heteroaryl (tritylsulfanyl group-containing aryl compounds) is synthesized in many cases by mainly reacting an aromatic thiol with triphenylmethanol or a trityl halide. A tritylsulfanyl group-containing aryl compound can also be synthesized by reacting an aromatic halide with tritylthiol through a cross-coupling reaction or the like, but this reaction is highly difficult. For example, in Non Patent Document 1, phenyl(trimethylsilyl)sulfide is synthesized by reacting phenyl iodide with triphenylmethanethiol, but this reaction is a reaction under harsh conditions of 110°C for 15 hours. Further, in Non Patent Document 2, phenyl(trimethylsilyl)sulfide is synthesized by reacting phenyl fluoride with triphenylmethanethiol, but this reaction took as long as 36 hours at room temperature. In Patent Document 4, a tritylsulfanyl group is introduced into imidazo[1,2-b]pyridazine by reacting chlorinated imidazo[1,2-b]pyridazine with triphenylmethanethiol, but the reaction needs to be carried out at 90°C for as long as 5 hours.

### Citation List

### Patent Documents

Patent Document 1: Published Japanese Translation No. 10-507206 of the PCT International Publication
Patent Document 2: Published Japanese Translation No. 2010-522213 of the PCT International Publication
Patent Document 3: International Patent Publication No. 2022/186304
Patent Document 4: International Patent Publication No. 1998/28299

### Non Patent Documents

Non Patent Document 1: Yoshida et al., European Journal of Organic Chemistry, 2014, vol. 19, p.3991-3995.
Non Patent Document 2: Satyanarayana et al., Chemical Communications, 2012, vol. 48, p.1461-1463.

### SUMMARY OF INVENTION

### Technical Problem

In a single-step method for synthesizing a pentafluorosulfanyl group-containing aryl compound using silver (II) fluoride and a tetraalkylammonium halide, it is expected that the pentafluorosulfanyl group-containing aryl compound can be synthesized more efficiently by optimizing a thioaryl compound used as a substrate.

An object of the present invention is to provide a method for producing a thioaryl compound suitable as a substrate for synthesizing a pentafluorosulfanyl group-containing aryl compound.

### Solution to Problem

The inventors of the present invention have found that a compound in which a tritylsulfanyl group is introduced into an aryl group or a heteroaryl group is suitable as a substrate for synthesizing a compound in which a pentafluorosulfanyl group is introduced into an aryl group or a heteroaryl group, since a tritylsulfanyl group bonded to an aryl group or a heteroaryl group is efficiently fluorinated by silver (II) fluoride and a tetraalkylammonium halide. Furthermore, they discovered that by using [(triphenylmethyl)sulfanyl]potassium or [(triphenylmethyl)sulfanyl]sodium, a halogen atom bonded to an aryl group or a heteroaryl group can be substituted with a tritylsulfanyl group under relatively mild conditions, and thus completed the present invention.

That is, the present invention is as follows.
[1] A method for producing a tritylsulfanyl group-containing aryl compound, the method including thiotritylation of a halogenated aryl compound represented by the following general formula (1) using [(triphenylmethyl)sulfanyl]potassium or [(triphenylmethyl)sulfanyl] sodium,
   [Chemical Formula 1]

   A¹-X (1)

   [wherein A¹ is an aryl group which may have a substituent or a heteroaryl group which may have a substituent; and X is a halogen atom]
   to produce a tritylsulfanyl group-containing aryl compound represented by the following general formula (2): 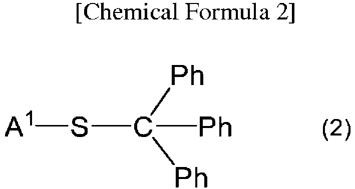 [wherein A¹ is the same as A¹ in the general formula (1), and Ph is a phenyl group].
[2] The method for producing a tritylsulfanyl group-containing aryl compound according to [1] above, further including thiotritylation of the halogenated aryl compound represented by the above general formula (1) using a palladium catalyst.
[3] The method for producing a tritylsulfanyl group-containing aryl compound according to [2] above, wherein the aforementioned palladium catalyst is Pd[cinnamyl](^{t}BuXPhos)OTf or Pd[allyl](AlPhos)OTf.
[4] The method for producing a tritylsulfanyl group-containing aryl compound according to any one of [1] to [3] above, wherein the aforementioned A¹ is
   an aryl group which may have one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a cyano group, a fluorinated formyl group, an amino group, a nitro group, a pyrrolidinyl group, a piperidinyl group, a piperazinyl group, a pyrazolidinyl group, an imidazolidinyl group, a tetrahydrofuranyl group, a 1,3-dioxolanyl group, a tetrahydrothiophenyl group, a 1,2-oxathiolanyl group, a morpholinyl group, and a tetrahydropyranyl group, or
   a heteroaryl group which may have one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a cyano group, a fluorinated formyl group, an amino group, a nitro group, a pyrrolidinyl group, a piperidinyl group, a piperazinyl group, a pyrazolidinyl group, an imidazolidinyl group, a tetrahydrofuranyl group, a 1,3-dioxolanyl group, a tetrahydrothiophenyl group, a 1,2-oxathiolanyl group, a morpholinyl group, and a tetrahydropyranyl group.
[5] The method for producing a tritylsulfanyl group-containing aryl compound according to any one of [1] to [4] above, wherein a reaction of the aforementioned thiotritylation is carried out at 0 to 80°C.
[6] A method for producing a pentafluorosulfanyl group-containing aryl compound, the method including:
   producing a tritylsulfanyl group-containing aryl compound represented by the above general formula (2) from a halogenated aryl compound represented by the above general formula (1) by the method for producing a tritylsulfanyl group-containing aryl compound according to any one of [1] to [5] above; and
   synthesizing a pentafluorosulfanyl group-containing aryl compound represented by the following general formula (3) from the aforementioned tritylsulfanyl group-containing aryl compound by an oxidative fluorination reaction using a divalent or higher metal fluoride and an organic salt containing a quaternary ammonium cation or a quaternary phosphonium cation
      [Chemical Formula 3]

      A¹-SF₅ (3)

      [wherein A¹ is the same as A¹ in the general formula (1)].
[7] The method for producing a pentafluorosulfanyl group-containing aryl compound according to [6] above, wherein the aforementioned oxidative fluorination reaction is carried out at -40 to 130°C.

### Advantageous Effects of Invention

The method according to the present invention makes it possible to introduce a tritylsulfanyl group into various aryl compounds under relatively mild conditions, and to efficiently synthesize a tritylsulfanyl group-containing aryl compound.

### DESCRIPTION OF EMBODIMENTS

In the present invention and the specification of the present application, "Cₚ₁₋ₚ₂" (p1 and p2 are positive integers satisfying p1 < p2) means a group in which the number of carbon atoms is from p1 to p2.

In the present invention and the specification of the present application, the term "C₁₋₆ alkyl group" is an alkyl group having 1 to 6 carbon atoms, and may be linear or branched, or may be cyclic. Examples of the C₁₋₆ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, and a cyclohexyl group.

In the present invention and the specification of the present application, the term "C₁₋₆ alkoxy group" refers to a group in which an oxygen atom is bonded to the bonding end of a C₁₋₆ alkyl group. The C₁₋₆ alkoxy group may be linear or branched. Examples of the C₁₋₆ alkoxy group include a methoxy group, an ethoxy group, a propoxy group, a butoxy group, a tert-butoxy group, a pentyloxy group, and a hexyloxy group.

In the present invention and the specification of the present application, the term "C₂₋₆ alkenyl group" refers to a group in which at least one carbon-carbon bond of an alkyl group having 2 to 6 carbon atoms is an unsaturated bond. The C₂₋₆ alkenyl group may be linear or branched, or may be cyclic. Examples of the C₂₋₆ alkenyl group include a vinyl group, an allyl group, a butenyl group, a pentenyl group, a hexenyl group, and a cyclohexenyl group.

In the present invention and the specification of the present application, the term "C₂₋₇ acyl group" refers to a group in which a hydrocarbon group moiety obtained by removing a carbonyl group from an acyl group is a C₁₋₆ alkyl group, a C₂₋₆ alkenyl group, a 5- or 6-membered aryl group, or a 5- or 6-membered heteroaryl group. The hydrocarbon group moiety of this acyl group may be linear or branched. Examples of the C₂₋₇ acyl group include a formyl group, an acetyl group, a propanoyl group, a propenoyl group, and a benzoyl group.

In the present invention and the specification of the present application, the term "halogen atom" refers to a fluorine atom, a chlorine atom, a bromine atom, or an iodine atom. A "halogen atom other than a fluorine atom" refers to a chlorine atom, a bromine atom, or an iodine atom. As an example of the "halogen atom other than a fluorine atom", a chlorine atom or a bromine atom is preferred, and a chlorine atom is particularly preferred.

Further, in the following, a "compound (n)" refers to a compound represented by a formula (n).

### <Method for producing tritylsulfanyl group-containing aryl compound>

The method for producing a tritylsulfanyl group-containing aryl compound (hereinafter sometimes referred to as an "STr-containing aryl compound") according to the present invention is a method for producing an STr-containing aryl compound represented by the following general formula (2) by thiotritylation of a halogenated aryl compound represented by the following general formula (1) using [(triphenylmethyl)sulfanyl]potassium or [(triphenylmethyl)sulfanyl]sodium.
[Chemical Formula 4]

A¹-X (1)

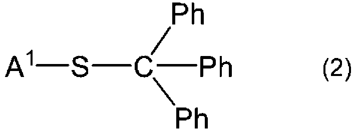

In the general formula (1), X is a halogen atom. As the halogenated aryl compound (1), X is preferably a fluorine atom, a chlorine atom, a bromine atom, or an iodine atom, and more preferably a chlorine atom or a bromine atom.

In the general formula (2), Ph represents an unsubstituted phenyl group (having no substituent).

In the general formulas (1) and (2), A¹ represents an aryl group which may have a substituent or a heteroaryl group which may have a substituent. In the present invention and the specification of the present application, the term "aryl group which may have a substituent" includes both an unsubstituted aryl group and an aryl group which has at least one substituent. Similarly, the term "heteroaryl group which may have a substituent" includes both an unsubstituted heteroaryl group and a heteroaryl group which has at least one substituent.

The aryl group and heteroaryl group represented by A¹ only need to be such that a ring structure to which X is bonded is a ring having aromaticity, and may be a group having a condensed ring of an aryl ring or heteroaryl ring and a non-aromatic ring. For example, as the aryl group represented by A¹, a monovalent group of a benzodioxane ring in which a benzene ring and a dioxane ring are condensed can be used.

When A¹ is an aryl group which may have a substituent, this aryl group is not particularly limited and examples thereof include a phenyl group, a naphthyl group, an anthryl group, and a 9-fluorenyl group, and a phenyl group is particularly preferred. When A¹ is a heteroaryl group which may have a substituent, this heteroaryl group is not particularly limited, and examples thereof include a pyridyl group, a pyrazinyl group, a pyrimidinyl group, a pyridazinyl group, a pyrazolyl group, a quinolyl group, an isoquinolyl group, a pyrrolyl group, an imidazolyl group, an indolyl group, a furyl group, a benzofuryl group, a thienyl group, a benzothienyl group, an oxazolyl group, a benzoxazolyl group, an isoxazolyl group, a benzoisoxazolyl group, a benzodioxazolyl group, a benzodioxolanyl group, a benzodioxanyl group, a quinazolinyl group, a quinoxalinyl group, a thiazolyl group, a benzothiazolyl group, an isothiazolyl group, a benzoisothiazolyl group, a thiazinyl group, a benzothiazinyl group, a benzothiophenyl group, a chromenyl group, a dibenzofuranyl group, a carbazolyl group, a fluorenyl group, a phenazinyl group, and a phenoxazinyl group.

An "aryl group having a substituent" refers to a group in which one or more, preferably 1 to 3, hydrogen atoms bonded to a carbon atom of the aryl group have been substituted with other functional groups. Similarly, a "heteroaryl group having a substituent" refers to a group in which one or more, preferably 1 to 3, hydrogen atoms bonded to a carbon atom of the heteroaryl group have been substituted with other functional groups. When two or more substituents are present, the substituents may be the same as or different from each other.

The substituent included in the aryl group or heteroaryl group represented by A¹ is not particularly limited as long as it does not inhibit the thiotritylation of the halogen atom of the halogenated aryl compound (1). Examples of the substituent include a halogen atom, an alkyl group, a fluorinated alkyl group, an alkenyl group, an alkoxy group, an aryl group, a heteroaryl group, an acyl group, a hydroxy group, a carboxy group, a cyano group, a fluoroformyl group (-C(=O)F), an amino group, a nitro group, and a non-aromatic heterocyclic group. The alkyl group is preferably a C₁₋₆ alkyl group, the alkenyl group is preferably a C₂₋₆ alkyl group, the alkoxy group is preferably a C₁₋₆ alkoxy group, and the acyl group is preferably a C₂₋₇ acyl group. The fluorinated alkyl group is preferably a group in which one or two or more hydrogen atoms of a C₁₋₆ alkyl group have been substituted with fluorine atoms, more preferably a fully fluorinated C₁₋₆ alkyl group in which all hydrogen atoms have been substituted with fluorine atoms, and particularly preferably a trifluoromethyl group. Examples of the aryl group and the heteroaryl group include the same groups as those listed as the aryl group and heteroaryl group represented by A¹, respectively, and a phenyl group or a pyridyl group is preferred. Examples of the non-aromatic heterocyclic group includes a pyrrolidinyl group, a piperidinyl group, a piperazinyl group, a pyrazolidinyl group, an imidazolidinyl group, a tetrahydrofuranyl group, a 1,3-dioxolanyl group, a tetrahydrothiophenyl group, a 1,2-oxathiolanyl group, a morpholinyl group, and a tetrahydropyranyl group.

When the substituent included in the aryl group or heteroaryl group represented by A¹ is an alkyl group, an alkenyl group, an alkoxy group, an aryl group, a heteroaryl group, an acyl group, or an amino group, these substituents may further have a substituent. Examples of this substituent include the same groups as those described above. For example, A¹ may be an aryl group having an unsubstituted C₁₋₆ alkyl group as a substituent, or an aryl group having a C₁₋₆ alkyl group in which one hydrogen atom has been substituted with a phenyl group as a substituent. Further, A¹ may be an aryl group having an unsubstituted amino group as a substituent, or an aryl group having an amino group in which one or two hydrogen atoms have been substituted with a phenyl group as a substituent.

The substituent included in the aryl group and heteroaryl group represented by A¹ may be protected with a protecting group. As the protecting group, a group commonly used in organic synthesis can be appropriately used. For example, when the substituent is an amino group, this amino group can have two hydrogen atoms substituted with a tert-butoxycarbonyl group, a benzyloxycarbonyl group, a 9-fluorenylmethyloxycarbonyl group, a 2,2,2-trichloroethoxycarbonyl group, an allyloxycarbonyl group, a trifluoroacetyl group, a phthaloyl group, a p-toluenesulfonyl group or a 2-nitrobenzenesulfonyl group before being subjected to a thiotritylation reaction. Similarly, when the substituent is a carboxy group, this carboxy group can have a hydrogen atom substituted with a benzyl group or a tert-butyl group.

When A¹ is a heteroaryl group which may have a substituent, the hetero atom in this heteroaryl group may also be protected with a protecting group. Examples of this protecting group include the same groups as those described above.

In the present invention, the halogenated aryl compound (1) is preferably one in which A¹ is a phenyl group which may have a substituent, a benzodioxazolyl group which may have a substituent, a benzodioxanyl group which may have a substituent, a dibenzofuranyl group which may have a substituent, an indolyl group which may have a substituent, a quinoxalinyl group which may have a substituent, a pyridyl group which may have a substituent, a pyrimidinyl group which may have a substituent, or a furanyl group which may have a substituent. Further, when these groups have a substituent, they are preferably a group having 1 to 3 substituents selected from the group consisting of a C₁₋₆ alkyl group which may have a substituent, a C₁₋₆ alkoxy group which may have a substituent, a C₂₋₇ acyl group which may have a substituent, a halogen atom, an amino group which may have a substituent, an aryl group which may have a substituent, a heteroaryl group which may have a substituent, a piperazinyl group which may have a substituent, and a cyano group.

In the present invention, the thiotritylation reaction of the halogenated aryl compound (1) uses [(triphenylmethyl)sulfanyl]potassium or [(triphenylmethyl)sulfanyl]sodium as an STr group source. In the present invention, by using a sodium salt or potassium salt of STr as the STr group source, the thiotritylation reaction can be carried out under relatively mild conditions, more specifically, at a reaction temperature within a range of 0 to 80°C.

In the present invention, the thiotritylation reaction of the halogenated aryl compound (1) can also be carried out using a catalyst. As the catalyst, a palladium catalyst is preferred, a catalyst composed of a palladium complex is more preferred, and a catalyst composed of a palladium complex in which a phosphine ligand is coordinated is still more preferred. The phosphine ligand is not particularly limited, and examples thereof include ^{t}BuXPhos (2-di-tert-butylphosphino-2',4',6'-triisopropylbiphenyl: CAS No. 564483-19-8), AlPhos (di-1-adamantyl(4"-butyl-2",3",5",6"-tetrafluoro-2',4',6'-triisopropyl-2-meoxy-meta-tert-phenyl)phosphine: CAS No. 1805783-60-1), Xphos (2-dicyclohexylphosphino-2',4',6'-triisopropylbiphenyl: CAS No. 564483-18-7), BrettPhos (2-(dicyclohexylphosphino)-3,6-dimethoxy-2',4',6'-triisopropyl-1,1'-biphenyl: CAS No. 1070663-78-3), Ad-BrettPhos (bis(adamantan-1-yl)[3,6-dimethoxy-2',4',6'-tris(propan-2-yl)-[1,1'-biphenyl]-2-yl]phosphane: CAS No. 1160861-59-5), Ad-BippyPhos (5-[di(adamantan-1-yl)phosphino]-1',3',5'-triphenyl-1'H-1,4'-bipyrazole: CAS No. 1239478-87-5), RuPhos (2-(dicyclohexylphosphino)-2',6'-isopropoxybiphenyl: CAS No. 787618-22-8), Me₃(OMe)^{t}BuXPhos (2-di-tert-butylphosphino-4-methoxy-3,5,6-trimethyl-2',4',6'-tri-i-propylbiphenyl: 792470-250MG, manufactured by Sigma-Aldrich Co. LLC.), Sphos (2-dicyclohexylphosphino-2',6'-dimethoxybiphenyl: CAS No. 657408-07-6), ^{t}Bu-BrettPhos (3,6-dimethoxy-2',4',6'-tris(1-methylethyl)[1,1'-biphenyl]-2-yl)bis(1,1-dimethylethyl)phosphine: CAS No. 1160861-53-9), Me₄^{t}BuXPhos (2-di-tert-butylphosphino-3,4,5,6-tetramethyl-2',4',6'-tri-i-propyl)-1,1'-biphenyl: CAS No. 857356-94-6), MePhos (2-dicyclohexylphosphino-2'-methylbiphenyl: CAS No. 251320-86-2), ^{t}Bu-BippyPhos (5-(di-tert-butylphosphino)-1',3',5'-triphenyl-1,4'-bi-1H-pyrazole: CAS No. 894086-00-1), JohnPhos ((2-biphenyl)di-tert-butylphosphine: CAS No. 224311-51-7), ^{t}BuDavePhos (2-di-tert-butylphosphino-2'-(N,N-dimethylamino)biphenyl: CAS No. 224311-49-3), XantPhos (4,5'-bis(diphenylphosphino)-9,9'-dimethylxanthene: CAS No. 161265-03-8), CyXantPhos (4,5-bis(dicyclohexylphosphino)-9,9-dimethylxanthene: CAS No. 940934-47-4), ^{t}BuXantPhos (2-di-tert-butylphosphino-2',4',6'-triisopropylbiphenyl: CAS No. 564483-19-8), and DPEPhos ((oxydi-2,1-phenylene)bis(diphenylphosphine): CAS No. 166330-10-5), and ^{t}BuXPhos, AlPhos, ^{t}Bu-BippyPhos, CyXantPhos, XantPhos, Ad-BrettPhos, Ad-BippyPhos, BrettPhos, and ^{t}Bu-BrettPhos are preferred. Examples of the catalyst composed of a palladium complex include Pd[cinnamyl](^{t}BuXPhos)OTf and Pd[allyl](AlPhos)OTf.

In the present invention, the amount of the STr group source ([(triphenylmethyl)sulfanyl]potassium or [(triphenylmethyl)sulfanyl]sodium) added to the reaction system of the thiotritylation reaction of the halogenated aryl compound (1) may be equal to or more than the stoichiometric amount. From the viewpoints of reaction efficiency and cost, the amount of [(triphenylmethyl)sulfanyl]potassium or [(triphenylmethyl)sulfanyl]sodium used in the thiotritylation reaction is preferably from 1 to 10 equivalents, and more preferably from 1 to 6 equivalents, of the halogenated aryl compound (1).

In the present invention, when a catalyst is used in the thiotritylation reaction of the halogenated aryl compound (1), the amount of the catalyst added to the reaction system is not particularly limited, but from the viewpoint of reaction efficiency, the concentration in the reaction system is preferably 0.1 mol% or more, more preferably 0.5 mol% or more, and still more preferably 1.0 mol% or more. Further, from the viewpoint of cost, the amount of catalyst used is preferably 20 mol% or less, more preferably 15 mol% or less, and still more preferably 10 mol% or less.

The thiotritylation reaction of the halogenated aryl compound (1) can be carried out in a solvent inert to the reaction. Although the inert solvent is not particularly limited, an aprotic polar solvent is preferred. Examples of the aprotic polar solvent include acetonitrile (MeCN), N,N'-dimethylformamide (DMF), N,N-dimethylacetamide, dimethylsulfoxide (DMSO), tetrahydrofuran (THF), dichloromethane (DCM), and diethyl ether. The solvent used in the reaction may be a mixed solvent of two or more solvents.

The thiotritylation reaction of the halogenated aryl compound (1) is carried out by reacting, at an appropriate temperature and time, a reaction solution obtained by mixing the halogenated aryl compound (1), an STr group source ([(triphenylmethyl)sulfanyl]potassium or [(triphenylmethyl)sulfanyl]sodium), and, if necessary, a catalyst in a reaction solvent. This thiotritylation reaction proceeds under relatively mild conditions. For example, the reaction temperature is not particularly limited as long as it is a temperature at which the reaction solvent is in a liquid state, and the reaction can be carried out at -40 to 130°C, is preferably carried out at 0 to 80°C, and particularly preferably carried out at 40 to 70°C, because this improves both the reaction efficiency and ease of reaction. For example, by carrying out the thiotritylation reaction at 0 to 80°C for 1 to 24 hours, the desired STr-containing aryl compound (2) can be obtained in an essentially quantitative yield.

### <Method for producing pentafluorosulfanyl group-containing aryl compound>

By oxidative fluorination of the above STr-containing aryl compound (2), a pentafluorosulfanyl group-containing aryl compound (SF₅-containing aryl compound) can be produced. This oxidative fluorination reaction can be carried out, for example, under the conditions described in Patent Document 3.

More specifically, the method for producing an SF₅-containing aryl compound according to the present invention produces an STr-containing aryl compound (2) from a halogenated aryl compound (1) by the above method for producing an STr-containing aryl compound, and synthesizes an SF₅-containing aryl compound represented by the following general formula (3) from the above tritylsulfanyl group-containing aryl compound by an oxidative fluorination reaction using a divalent or higher valent metal fluoride and an organic salt containing a quaternary ammonium cation or a quaternary phosphonium cation. In the general formula (3), A¹ is the same as A¹ in the general formula (1).
[Chemical Formula 5]

A¹-SF₅ (3)

Examples of the divalent or higher valent metal fluoride used as a catalyst include a fluoride of a first transition element, a second transition element, or a third transition element. As a fluorinating agent used in the present invention, more specifically, a divalent or higher valent fluoride of silver, niobium, manganese, cobalt, copper, hafnium, tantalum, or cerium is preferred, and examples thereof include AgF₂, manganese (III) fluoride (MnF₃), cobalt (III) fluoride (CoF₃), copper (II) fluoride (CuF₂), niobium (V) fluoride (NbF₅), hafnium (V) fluoride (HfF₅), tantalum fluoride (TaF₅), and cerium (IV) fluoride (CeF₄). AgF₂ is particularly preferred as a divalent or higher valent metal fluoride used as a fluorinating agent in the present invention from the viewpoint of favorable reactivity.

The organic salt used as a fluorinating agent in the above oxidative fluorination reaction is not particularly limited as long as it is an organic salt containing a quaternary ammonium cation or a quaternary phosphonium cation. Examples of the organic salt include compounds represented by the following general formulas (s1) to (s7).

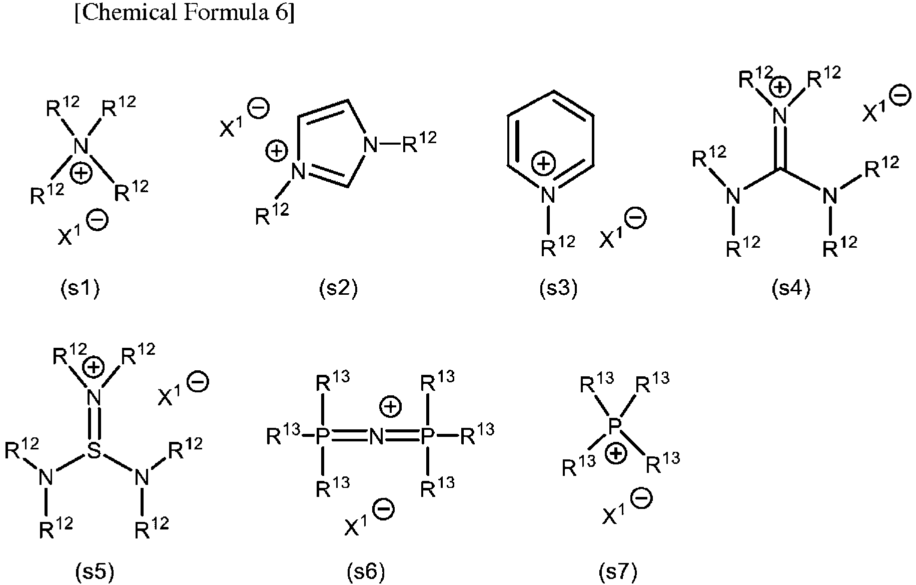

In the general formulas (s1) to (s5), R¹² is a C₁₋₆ alkyl group or an aryl group. This aryl group can be the same group as those mentioned above for A¹. A plurality of R¹² groups in one molecule may all be the same group or may be different groups from each other.

In the general formulas (s6) to (s7), R¹³ is a C₁₋₆ alkyl group, an aryl group, a C₁₋₆ alkoxy group, or a C₁₋₆ alkylamino group. This aryl group can be the same groups as those mentioned above for A¹. The C₁₋₆ alkylamino group is not particularly limited as long as it is a group in which one or two hydrogen atoms of an amino group are substituted with a C₁₋₆ alkyl group. Examples of the C₁₋₆ alkylamino group include a dimethylamino group. A plurality of R¹³ groups in one molecule may all be the same group or may be different groups from each other.

In the general formulas (s1) to (s7), X¹ is not particularly limited as long as it is a monovalent anion that forms a salt with a quaternary ammonium cation or a quaternary phosphonium cation. Examples of this X¹⁻ include iodine ions (I⁻), bromide ions (Br⁻), chloride ions (Cl⁻), fluoride ions (F⁻), hydrogen difluoride ions (HF₂⁻), tribromide ions (Br₃⁻), azide ions (N₃⁻), cyanide ions (CN⁻), and cyanate ions (OCN⁻).

As the organic salt used as the fluorinating agent in the above oxidative fluorination reaction, a tetraalkylammonium halide (NR¹¹₄X) is particularly preferred. The NR¹¹₄X used in the above oxidative fluorination reaction is not particularly limited as long as it is a halide in which four alkyl groups are bonded to a nitrogen atom. As the halide, a chloride or a bromide is preferred, and a chloride is particularly preferred. Further, the alkyl group bonded to the nitrogen atom may be linear or branched, and the four alkyl groups may all be the same group or may be different groups from each other. The alkyl group is preferably a C₁₋₆ alkyl group, and more preferably a methyl group, an ethyl group, or a propyl group. Among them, NR¹¹₄X is preferably N(Et)₄Cl (tetraethylammonium chloride) (CAS No: 56-34-8) or N(Et)₄Br (tetraethylammonium bromide) (CAS No: 71-91-0), and more preferably N(Et)₄Cl.

The amount of the organic salt to be added to the reaction system such as NR¹¹₄X may be equal to or more than the stoichiometric amount. From the viewpoints of reaction efficiency and cost, the amount of the organic salt such as NR¹¹₄X used in the above oxidative fluorination reaction is preferably from 1 to 10 equivalents, and more preferably from 1 to 6 equivalents of the STr-containing aryl compound (2).

Although the amount of the divalent or higher valent metal fluoride to be added to the reaction system such as AgF₂ is not particularly limited, from the viewpoint of reaction efficiency, it is preferably 5 equivalents or more, more preferably 8 equivalents or more, and still more preferably 10 equivalents or more of the STr-containing aryl compound (2). In addition, from the viewpoint of cost, the amount of the divalent or higher valent metal fluoride such as AgF₂ used in the above oxidative fluorination reaction is preferably 100 equivalents or less, more preferably 50 equivalents or less, still more preferably 30 equivalents or less, and even more preferably 20 equivalents or less of the STr-containing aryl compound (2).

The above oxidative fluorination reaction can be carried out in a solvent inert to the reaction. Although the inert solvent is not particularly limited, an aprotic polar solvent is preferred. As the aprotic polar solvent, those mentioned above can be used. The solvent used in the reaction may be a mixed solvent of two or more solvents.

The above oxidative fluorination reaction is carried out by reacting, at an appropriate temperature and time, a reaction solution obtained by mixing an STr-containing aryl compound (2), an organic salt such as NR¹¹₄X, and a divalent or higher valent metal fluoride such as AgF₂ in a reaction solvent. This oxidative fluorination reaction proceeds under mild conditions. For example, the reaction temperature is not particularly limited as long as it is a temperature at which the reaction solvent is in a liquid state, and the reaction can be carried out at -40 to 130°C, is preferably carried out at 0 to 80°C, and can also be carried out at room temperature (0 to 30°C). For example, by carrying out the oxidative fluorination reaction at room temperature for less than 1 hour, the desired SF₅-containing aryl compound (3) can be obtained in an essentially quantitative yield.

By the above oxidative fluorination reaction, the divalent or higher valent metal fluoride such as AgF₂ is defluorinated to produce a metal such as Ag. By recovering and fluorinating the metal such as Ag produced by the reaction, a divalent or higher valent metal fluoride such as AgF₂ can be regenerated. The regenerated divalent or higher valent metal fluoride such as AgF₂ can be used again in the above oxidative fluorination reaction. Fluorination of a metal such as Ag can be carried out, for example, by a conventional method such as heating in fluorine gas.

The above oxidative fluorination reaction enables the one-pot synthesis of the SF₅-containing aryl compound (3) in a single step under relatively mild reaction conditions in a one-pot manner with a high yield. Further, in this oxidative fluorination reaction, a partially fluorinated product such as an SF₄Cl group-containing aryl compound is not produced in most cases. For this reason, there is also an advantage that it is not necessary to isolate an SF₄Cl group-containing aryl compound from the reaction product and purify the desired SF₅-containing aryl compound (3).

### Examples

Hereinafter, the present invention will be described with reference to Examples, but the present invention is not limited to these Examples.

Unless otherwise stated, all reagents were purchased from commercial suppliers and used without further purification.

Dried and degassed solvents were used in all reactions under argon. All reported yields refer to spectroscopically and chromatographically pure compounds unless otherwise specified.

Analytical thin layer chromatography (TLC) was performed on a glass plate precoated with silica gel (layer thickness: 0.25 nm, Kiesel gel 60 F254, manufactured by Merck KGaA) and visualized with a UV lamp (254 or 365 nm) and/or an appropriate staining reagent.

Column chromatography was performed using Kanto Silica Gel 60N (spherical, neutral).

¹H, ¹³C, ¹⁹F, and ³¹P nuclear magnetic resonance (NMR) spectra were measured at ambient temperature using a JNM-ECZ400S NMR instrument (manufactured by JEOL Ltd.) or an ECZ500R NMR instrument (manufactured by JEOL Ltd.) unless otherwise specified. The solvents used and the respective measurement frequencies are indicated for each experiment. Resonance multiplicities are expressed as s (singlet), d (doublet), t (triplet), q (quadruplet), p (pentet), m (multiplet), and br (broad). Residual deuterated solvent signals relative to tetramethylsilane (for example, CDCl₃ = 7.26 ppm for ¹H and 77.160 ppm for ¹³C) were used as internal references for ¹H and ¹³C NMR spectra and reported as chemical shifts in ppm (multiplicity, coupling constant J in Hz, number of protons).

All spectra were broadband decoupled unless otherwise noted.

### [Synthesis Example 1] Synthesis of Pd[cinnamyl](^{t}BuXPhos)OTf catalyst (catalyst 1)

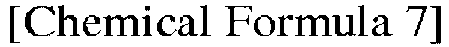

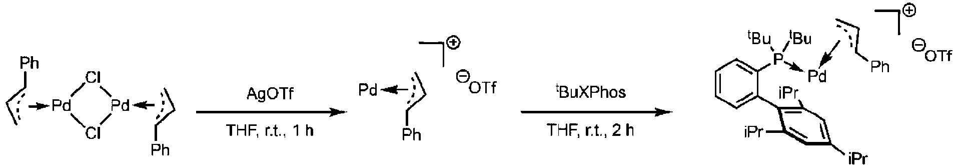

In a 15 mL glass vial in a glove box under an argon atmosphere, silver trifluoromethanesulfonate (AgOTf, 308.3 mg, 1.2 mmol, 2.0 equivalents) was added to a stirred mixture of THF (6.0 mL) and Pd[cinnamyl]Cl dimer (310.8 mg, 0.6 mmol, 1.0 equivalent). The resulting mixture in which silver chloride was formed as a white precipitate was stirred at room temperature for 1 hour. The white precipitate in the reaction mixture was removed with a syringe filter, and the filtrate was added to a 50 mL glass vial containing ^{t}BuXPhos (509.6 mg, 1.2 mmol, 2.0 equivalents). After stirring at room temperature for 2 hours, hexane (30 mL) was added to the reaction solution to obtain an orange precipitate. The supernatant was removed by decantation, and the residue was washed three times with hexane (10 mL). The residue was evaporated under reduced pressure to obtain Pd[cinnamyl](^{t}BuXPhos)OTf (937.3 mg, yield: 98%) as an orange powder.

¹H NMR (400 MHz, CDCl₃) δ 7.96 (t, J = 7.2 Hz, 1H), 7.54 (t, J = 8.0 Hz, 2H), 7.47 (tt, J = 7.3, 1.5 Hz, 1H), 7.35 (t, J = 7.7 Hz, 2H), 7.13 (br, 3H), 6.82 - 6.62 (m, 1H), 5.71 (br, 1H), 4.91 (br, 1H), 2.62 - 2.10 (m, 2H), 1.67 - 0.71 (m, 39H).

¹⁹F NMR (376 MHz, CDCl₃) δ -78.0.

¹³C NMR (101 MHz, CDCl₃) δ 153.1, 151.7, 147.1, 146.8, 135.6, 135.4, 135.1, 133.8, 133.7, 131.6, 131.5, 130.4 (two peaks), 129.8 (two peaks), 128.1, 128.0, 125.4, 123.3, 122.7, 119.5, 119.0, 116.3, 110.2, 39.4, 39.3, 32.3, 31.5, 31.4 (two peaks), 31.1, 25.9, 25.7, 25.1, 24.9, 24.6, 22.8, 22.5, 14.3. [observed complexity due to C-P and C-F coupling].

³¹P NMR (162 MHz, CDCl₃) δ 76.7.

### [Synthesis Example 2] Synthesis of Pd[allyl](AlPhos)OTf catalyst (catalyst 2)

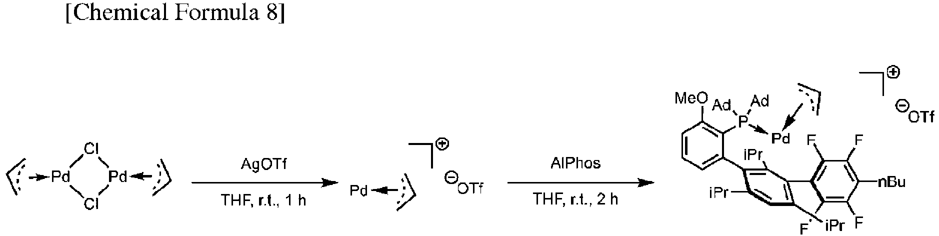

In a 15 mL glass vial in a glove box under an argon atmosphere, silver trifluoromethanesulfonate (AgOTf, 25.7 mg, 0.1 mmol, 2.0 equivalents) was added to a stirred mixture of THF (1.0 mL) and allylpalladium chloride dimer (18.3 mg, 0.05 mmol, 1.0 equivalent). The resulting mixture in which silver chloride was formed as a white precipitate was stirred at room temperature for 1 hour. The white precipitate in the reaction mixture was removed with a syringe filter, and the filtrate was added to a 50 mL glass vial containing AlPhos (81.5 mg, 0.1 mmol, 2.0 equivalents). After stirring at room temperature for 2 hours, hexane (10 mL) was added to the reaction solution to obtain an orange precipitate. The supernatant was removed by decantation, and the residue was washed three times with hexane (5 mL). The residue was evaporated under reduced pressure to obtain Pd[allyl](AlPhos)OTf (108.5 mg, yield: 98%) as an orange powder.

¹H NMR (400 MHz, CDCl₃) δ 7.75 (d, J = 12.9 Hz, 1H), 7.53 (t, J = 8.0 Hz, 1H), 7.19 - 7.10 (m, 1H), 6.38 - 6.24 (m, 1H), 6.09 - 5.83 (m, 1H), 5.75 - 5.49 (m, 1H), 4.73 (dd, J = 61.4, 6.1 Hz, 1H), 4.03 (s, 3H), 3.89 (dd, J = 13.6, 9.0 Hz, 1H), 3.48 (dd, J = 14.3, 8.2 Hz, 1H), 3.05 (dd, J = 23.9, 12.1 Hz, 2H), 2.84 (t, J = 7.4 Hz, 2H), 2.77 - 2.60 (m, 1H), 2.52 - 1.92 (m, 20H), 1.70 (m, 14H), 1.57 (s, 1H), 1.44 (m, 4H), 1.32-1.14 (m, 6H), 1.07 - 0.95 (m, 4H), 0.90 (t, J = 6.5 Hz, 4H), 0.80 (d, J = 6.7 Hz, 1H), 0.69 (d, J = 5.9 Hz, 1H).

¹⁹F NMR (376 MHz, CDCl₃) δ -78.0 (s, 3F), -135.5 - -136.2 (m), -136.8 (dd, J = 23.8, 12.3 Hz), -138.6 (dd, J = 23.9, 12.1 Hz), -142.9 (ddd, J = 98.4, 23.6, 12.3 Hz), - 143.7 (ddd, J = 45.7, 23.8, 12.4 Hz).

¹³C NMR (101 MHz, CDCl₃) δ 161.8 (dd, J = 3.6, 2.2 Hz), 155.1, 154.6, 154.5, 153.8, 152.0, 148.4 (dd, J = 24.9, 21.3 Hz), 146.0, 134.0, 127.5, 127.3 (d, J = 11.2 Hz), 126.9, 126.01, 122.7, 122.6 - 121.3 (m), 120.2 (d, J = 3.8 Hz), 119.5, 118.7, 111.5 (d, J = 2.6 Hz), 102.0 (dd, J = 25.1, 6.4 Hz), 98.0 (dd, J = 26.6, 10.1 Hz), 68.0, 60.4, 57.4, 54.9, 45.8 (dd, J = 11.5, 2.0 Hz), 44.9 (dd, J = 36.3, 10.3 Hz), 43.1 - 40.9 (m), 36.2 (d, J = 8.3 Hz), 34.2, 32.9 (dd, J = 60.7, 51.0 Hz), 31.4, 30.9, 30.0 - 28.3 (m), 26.1, 25.9 - 25.6 (m), 25.5, 24.3 (d, J = 11.1 Hz), 23.9, 23.8, 23.2, 22.9, 22.4, 22.4 - 22.3 (m), 21.5 (d, J = 3.1 Hz), 14.1, 13.8. [observed complexity due to C-P and C-F coupling].

³¹P NMR (162 MHz, CDCl₃) δ 88.2 (d, J = 101.6 Hz).

### [Synthesis Example 3] Synthesis of [(triphenylmethyl)sulfanyl]potassium or [(triphenylmethyl)sulfanyl] sodium

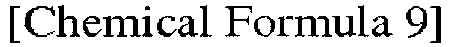

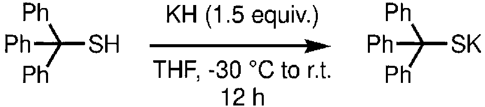

In a 100 mL round-bottom flask in a glove box under an argon atmosphere, triphenylmethanethiol (8.29 g, 30 mmol, 1.0 equivalent) was dissolved in THF (60 mL). The obtained solution was cooled to -30°C and potassium hydride (KH, 1.80 g, 45 mmol, 1.5 equivalents) was gradually added with vigorous stirring. Subsequently, the flask was removed from a cold bath, and the resulting mixture was stirred vigorously at room temperature for 12 hours. The reaction mixture was then filtered to remove excess KH and concentrated under reduced pressure to obtain [(triphenylmethyl)sulfanyl]potassium (KSTr) (9.34 g, yield: 99%) as a yellow powder.

¹H NMR (500 MHz, THF-d8) δ 7.55 (dd, J = 7.5, 0.9 Hz, 6H), 6.98 (t, J = 7.7 Hz, 6H), 6.90 - 6.85 (m, 3H).

By carrying out the reaction in the same manner using sodium hydride instead of potassium hydride, [(triphenylmethyl)sulfanyl]sodium (NaSTr) (8.95 g, yield: 99%) was obtained.

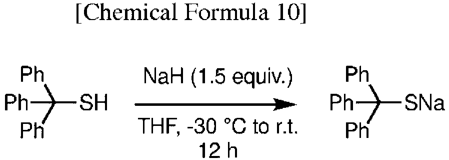

### [Synthesis Example 4] Synthesis of Pd[cinnamyl](DPEPhos)OTf catalyst (catalyst 3)

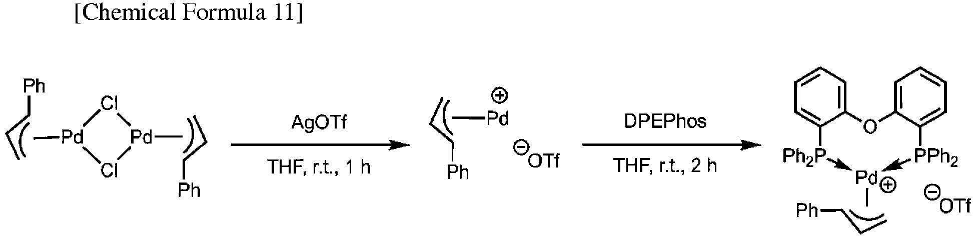

In a 15 mL glass vial in a glove box under an argon atmosphere, silver trifluoromethanesulfonate (AgOTf, 256.9 mg, 1.0 mmol, 2.0 equivalents) was added to a stirred mixture of THF (10.0 mL) and Pd[cinnamyl]Cl dimer (259.0 mg, 0.5 mmol, 1.0 equivalent). The resulting mixture in which silver chloride was formed as a white precipitate was stirred at room temperature for 1 hour. The white precipitate in the reaction mixture was removed with a syringe filter, and the filtrate was added to a 50 mL glass vial containing DPEPhos (538.6 mg, 1.0 mmol, 2.0 equivalents). After stirring at room temperature for 2 hours, hexane (30 mL) was added to the reaction solution to obtain an orange precipitate. The supernatant was removed by decantation, and the residue was washed three times with hexane (10 mL). The residue was evaporated under reduced pressure to obtain Pd[cinnamyl](DPEPhos)OTf (907.1 mg, yield: 99%) as an orange powder.

¹H NMR (400 MHz, DMSO-d₆) δ 7.68 - 7.01 (m, 24H), 6.96 - 6.78 (m, 6H), 6.69 (t, J = 7.6 Hz, 1H), 6.61 (dd, J = 8.1, 4.7 Hz, 2H), 6.08 (t, J = 7.8 Hz, 1H), 5.43 (br, 1H), 3.90 (t, J = 11.6 Hz, 1H).

¹⁹F NMR (376 MHz, CDCl₃) δ -77.7.

### [Production Example 1] Typical production method A for synthesizing aryl trityl sulfide compounds (tritylsulfanyl group-containing aryl compounds)

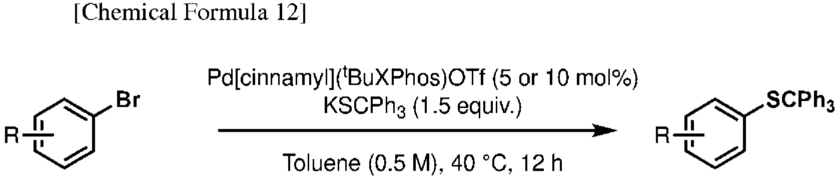

A test tube dried in an oven was moved into a glove box under an argon atmosphere. After adding aryl bromide (0.2 mmol) to a mixture of the catalyst 1 (8.0 mg, 5 mol%) and toluene (0.4 mL, 0.5 M) in this test tube, the test tube was cooled in a refrigerator at -30°C for 0.5 hours. KSTr (KSCPh₃) (94.3 mg, 0.3 mmol, 1.5 equivalents) was added to this mixture with vigorous stirring at room temperature. After sealing with an aluminum cap with a septum using a vial crimper, the test tube was taken out of the glove box, and the resulting mixture was stirred at 40°C (oil bath) for 12 hours. The solvent of the reaction mixture was removed by a rotary evaporator, and the crude material was purified by silica column chromatography to obtain the corresponding aryl trityl sulfide.

### [Production Example 2] Typical production method B for synthesizing aryl trityl sulfide compounds

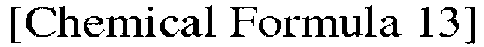

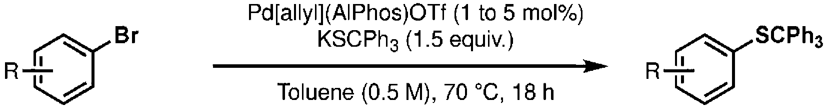

A test tube dried in an oven was moved into a glove box under an argon atmosphere. After adding aryl bromide to a mixture of the catalyst 2 and toluene (0.5 M) in this test tube, the test tube was cooled in a refrigerator at -30°C for 0.5 hours. KSTr (1.5 equivalents) was added to this mixture with vigorous stirring at room temperature. After sealing with an aluminum cap with a septum using a vial crimper, the test tube was taken out of the glove box, and the resulting mixture was stirred at 70°C (oil bath) for 18 hours. The solvent of the reaction mixture was removed by a rotary evaporator, and the crude material was purified by silica column chromatography to obtain the corresponding aryl trityl sulfide.

### [Production Example 3] Typical production method C for synthesizing aryl trityl sulfide compounds

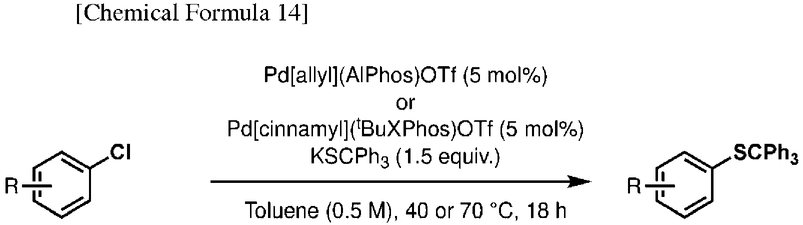

A test tube dried in an oven was moved into a glove box under an argon atmosphere. After adding aryl chloride to a mixture of the catalyst 1 or catalyst 2 (5 mol%) and toluene (0.5 M) in this test tube, the test tube was cooled in a refrigerator at - 30°C for 0.5 hours. KSTr (1.5 equivalents) was added to this mixture with vigorous stirring at room temperature. After sealing with an aluminum cap with a septum using a vial crimper, the test tube was taken out of the glove box, and the resulting mixture was stirred at 40°C (in the case of catalyst 1) or 70°C (in the case of catalyst 2) (oil bath) for 18 hours. The reaction mixture was diluted with CDCl₃, and the yield of the aryl chloride raw material was determined by ¹H NMR.

### [Example 1]

### 4-Methoxyphenyltrityl sulfide was synthesized by the production method A described in Production Example 1, or a production method A' using NaSTr.

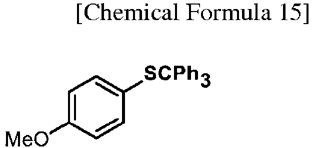

### (1) Production Method A

It was prepared from 4-bromoanisole (1.0 mmol, 187.0 mg) using the catalyst 1 (5 mol%, 39.9 mg) in accordance with the production method A. After the reaction, purification was carried out by silica column chromatography (hexane/EtOAc = 40/1 → 20/1 → 10/1 (volume ratio)) to obtain 4-methoxyphenyltrityl sulfide (369.9 mg, yield: 97%) as a white powder.

¹H NMR (400 MHz, CDCl₃) δ 7.41 - 7.36 (m, 6H), 7.25 - 7.15 (m, 9H), 6.91 - 6.85 (m, 2H), 6.58 - 6.52 (m, 2H), 3.70 (s, 3H).

¹³C{¹H} NMR (101 MHz, CDCl₃) δ 160.2, 144.8, 137.9, 130.1, 127.7, 126.7, 124.4, 113.8, 70.9, 55.3.

### (2) Production Method A' (production method using NaSTr)

A reaction mixture containing 4-methoxyphenyltrityl sulfide was obtained in the same manner as the production method A, with the exception that 4-bromoanisole (0.2 mmol, 37.4 mg) was used as the aryl bromide and NaSTr (NaSCPh₃) (89.5 mg, 0.3 mmol, 1.5 equivalents) was used instead of KSTr. The obtained reaction mixture was diluted with CDCl₃, and 1,4-bis(trifluoromethyl)benzene (15.5 µL, 0.1 mmol) was added as an internal standard to determine the NMR yield of 4-methoxyphenyltrityl sulfide, which was 61%.

Compared to the NMR yield of the reaction carried out using KSCPh₃ (NMR yield: 99%), from the viewpoints of reaction efficiency and cost, it was preferable that the STr group source used in the thiotritylation reaction was

### [(triphenylmethyl)sulfanyl]potassium.

### [Example 2]

3,5-Dimethoxyphenyltrityl sulfide was synthesized by the production method A described in Production Example 1.

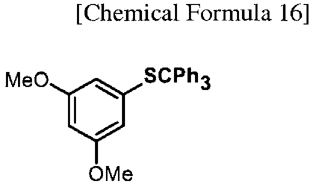

More specifically, it was prepared from 1-bromo-3,5-dimethoxybenzene (0.2 mmol, 43.4 mg) and the catalyst 1 (10 mol%, 16.0 mg) in accordance with the production method A. After the reaction, 3,5-dimethoxyphenyltrityl sulfide (66.1 mg, yield: 80%) was obtained as a white powder through purification by silica column chromatography (hexane/DCM = 10:1 (volume ratio)).

¹H NMR (400 MHz, CDCl₃) δ 7.41 - 7.37 (m, 6H), 7.27 - 7.18 (m, 9H), 6.22 (t, J = 2.3 Hz, 1H), 6.14 (d, J = 2.3 Hz, 2H), 3.49 (s, 6H).

¹³C{¹H} NMR (101 MHz, CDCl₃) δ 160.0, 144.6, 136.7, 130.2, 127.8, 126.9, 111.4, 101.2, 70.7, 55.3.

### [Example 3]

3,4,5-Trimethoxyphenyl trityl sulfide was synthesized by the production method A described in Production Example 1.

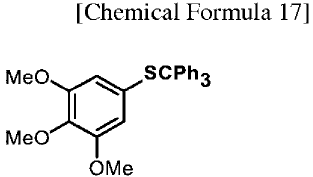

More specifically, it was prepared from 5-bromo-1,2,3-trimethoxybenzene (0.2 mmol, 49.4 mg) and the catalyst 1 (5 mol%, 8.0 mg) in accordance with the production method A. After the reaction, purification was carried out by silica column chromatography (hexane/EtOAc = 100/1 → 40/1 → 20/1 (volume ratio)) to obtain 3,4,5-trimethoxyphenyltrityl sulfide (67.2 mg, yield: 76%) as a white powder.

¹H NMR (400 MHz, CDCl₃) δ 7.41 - 7.34 (m, 6H), 7.27 - 7.18 (m, 9H), 6.21 (s, 2H), 3.77 (s, 3H), 3.50 (s, 6H).

¹³C{¹H} NMR (101 MHz, CDCl₃) δ 152.5, 144.6, 138.5, 130.2, 128.7, 127.8, 126.9, 112.7, 71.0, 61.0, 56.0.

### [Example 4]

6-[(trityl)thio]-1,4-benzodioxane was synthesized by the production method A described in Production Example 1.

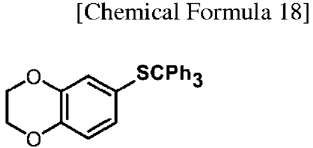

More specifically, it was prepared from 6-bromo-1,4-benzodioxane (0.2 mmol, 43.0 mg) and the catalyst 1 (5 mol%, 8.0 mg) in accordance with the production method A. After the reaction, purification was carried out by silica column chromatography (hexane/EtOAc = 10/1 → 5/1 (volume ratio)) to obtain 6-[(trityl)thio]-1,4-benzodioxane (68.2 mg, yield: 83%) as a white powder.

¹H NMR (400 MHz, CDCl₃) δ 7.43 - 7.37 (m, 6H), 7.26 - 7.15 (m, 9H), 6.52 - 6.42 (m, 3H), 4.19 - 4.10 (m, 4H).

¹³C NMR (101 MHz, CDCl₃) δ 144.8, 144.3, 142.9, 130.1, 129.6, 127.7, 126.7, 125.5, 125.0, 116.9, 71.0, 64.5, 64.2.

### [Example 5]

4-Chlorophenyltrityl sulfide was synthesized by the production method A described in Production Example 1.

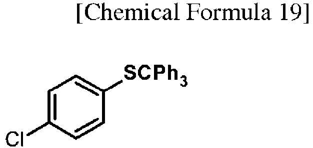

More specifically, it was prepared from 1-bromo-4-chlorobenzene (0.2 mmol, 38.3 mg) and the catalyst 1 (5 mol%, 8.0 mg) in accordance with the production method A. After the reaction, purification was carried out by silica column chromatography (hexane/DCM = 20/1 (volume ratio)) to obtain 4-chlorophenyltrityl sulfide (51.8 mg, yield: 67%) as a yellow powder.

¹H NMR (400 MHz, CDCl₃) δ 7.39 (ddd, J = 6.0, 2.2, 0.9 Hz, 6H), 7.29 - 7.18 (m, 9H), 6.98 - 6.94 (m, 2H), 6.89 - 6.84 (m, 2H).

### [Example 6]

4-Fluorophenyltrityl sulfide was synthesized by the production method A described in Production Example 1.

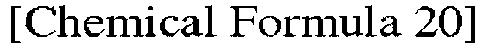

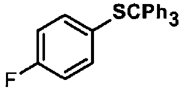

More specifically, it was prepared from 4-bromofluorobenzene (0.2 mmol, 35.0 mg) and the catalyst 1 (5 mol%, 8.0 mg) in accordance with the production method A. After the reaction, purification was carried out by silica column chromatography (hexane/EtOAc = 100/1 → 40/1 → 20/1 (volume ratio)) to obtain 4-fluorophenyltrityl sulfide (66.8 mg, yield: 90%) as a yellow powder.

¹H NMR (500 MHz, CDCl₃) δ 7.41 - 7.38 (m, 6H), 7.25 - 7.17 (m, 9H), 6.94 - 6.90 (m, 2H), 6.72 - 6.67 (m, 2H).

¹³C{^{{1}H} NMR (126 MHz, CDCl₃) δ 163.0 (d, 1JC-F = 248.8 Hz), 144.5, 137.6 (d, 3JC-F = 8.4 Hz), 130.0, 127.8, 126.8, 115.3 (d, 2JC-F = 21.5 Hz), 71.1.

¹⁹F NMR (471 MHz, CDCl₃) δ -112.8.

### [Example 7]

2-[(trityl)thio)-dibenzofuran was synthesized by the production method A described in Production Example 1.

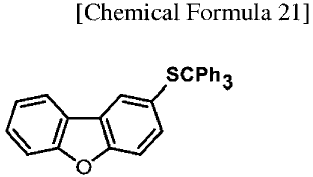

More specifically, it was prepared from 2-bromodibenzofuran (0.2 mmol, 49.4 mg) and the catalyst 1 (5 mol%, 8.0 mg) in accordance with the production method A. After the reaction, purification was carried out by silica column chromatography (hexane/EtOAc = 40/1 → 20/1 → 10/1 (volume ratio)) to obtain 2-[(trityl)thio)-dibenzofuran (86.3 mg, yield: 97%) as a white powder.

¹H NMR (400 MHz, CDCl₃) δ 7.67 (ddd, J = 7.7, 1.3, 0.6 Hz, 1H), 7.52 - 7.35 (m, 9H), 7.30 - 7.13 (m, 12H).

¹³C{¹H} NMR (101 MHz, CDCl₃) δ 156.5, 156.4, 144.7, 135.3, 130.2, 128.9, 128.1, 127.8, 127.4, 126.8, 124.5, 123.9, 123.0, 120.7, 111.8, 111.4, 71.3.

### [Example 8]

5'-[(trityl)thio]-m-terphenyl was synthesized by the production method A described in Production Example 1.

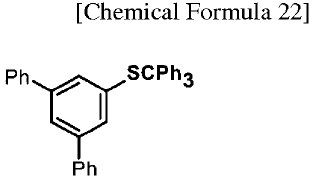

More specifically, it was prepared from 5'-bromo-m-terphenyl (0.2 mmol, 61.8 mg) and the catalyst 1 (5 mol%, 8.0 mg) in accordance with the production method A. After the reaction, purification was carried out by silica column chromatography (hexane/DCM = 20/1 → 10/1 → 5/1 (volume ratio)) to obtain 5'-[(trityl)thio]-m-terphenyl (88.7 mg, yield: 88%) as a white powder.

¹H NMR (400 MHz, CDCl₃) δ7.56 (t, J = 1.7 Hz, 1H), 7.44 (m, 6H), 7.40 - 7.19 (m, 21H).

¹³C{¹H} NMR (101 MHz, CDCl₃) δ 144.6, 141.6, 140.5, 135.8, 132.0, 130.3, 128.8, 127.9, 127.6, 127.2, 127.0, 125.5, 71.0.

### [Example 9]

1-[(trityl)thio]-4-(trans-4'-propylcyclohexyl)benzene was synthesized by the production method A described in Production Example 1.

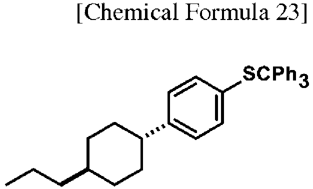

More specifically, it was prepared from 1-bromo-4-(trans-4-propylcyclohexyl)benzene (0.5 mmol, 140.6 mg) and the catalyst 1 (5 mol%, 19.9 mg) in accordance with the production method A. After the reaction, purification was carried out by silica column chromatography (hexane/DCM = 0/1 → 50/1 → 20/1 → 10/1 (volume ratio)) to obtain 1-[(trityl)thio]-4-(trans-4'-propylcyclohexyl)benzene (234.5 mg, yield: 98%) as a white powder.

¹H NMR (500 MHz, CDCl₃) δ 7.45 - 7.36 (m, 6H), 7.24 - 7.13 (m, 9H), 6.88 - 6.80 (m, 4H), 2.38 - 2.23 (m, 1H), 1.78 (t, J = 15.0 Hz, 4H), 1.40 - 1.13 (m, 7H), 1.05 - 0.92 (m, 2H), 0.88 (t, J = 7.2 Hz, 3H).

¹³C{¹H} NMR (101 MHz, CDCl₃) δ 148.2, 144.9, 135.4, 130.9, 130.2, 127.7, 126.8, 126.7, 70.9, 44.4, 39.8, 37.1, 34.3, 33.6, 20.2, 14.5.

### [Example 10]

4-[(trityl)thio]-4'-(diphenylamino)biphenyl was synthesized by the production method A described in Production Example 1.

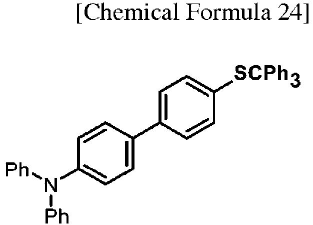

More specifically, it was prepared from 4-bromo-4'-(diphenylamino)biphenyl (0.2 mmol, 80.1 mg) and the catalyst 1 (5 mol%, 8.0 mg) in accordance with the production method A. After the reaction, purification was carried out by silica column chromatography (hexane/EtOAc = 100/1 (volume ratio)) to obtain 4-[(trityl)thio]-4'-(diphenylamino)biphenyl (101.0 mg, yield: 85%) as a white powder.

¹H NMR (500 MHz, CDCl₃) δ 7.44 - 7.41 (m, 6H), 7.38 - 7.35 (m, 2H), 7.28 - 7.17 (m, 15H), 7.12 - 7.06 (m, 6H), 7.05 - 6.97 (m, 4H).

¹³C{¹H} NMR (101 MHz, CDCl₃) δ 147.7, 147.5, 144.7, 140.0, 135.1, 134.1, 132.9, 130.2, 129.4, 127.8, 127.6, 126.8, 126.2, 124.6, 123.8, 123.2, 71.0.

### [Example 11]

tert-butyl 4-{4-[(trityl)thio]phenyl} piperazine-1-carboxylate was synthesized by the production method A described in Production Example 1.

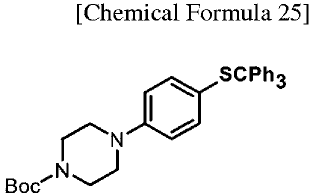

More specifically, it was prepared from tert-butyl 4-(4-bromophenyl)piperazine-1-carboxylate (0.2 mmol, 68.2 mg) and the catalyst 1 (5 mol%, 8.0 mg) in accordance with the production method A. After the reaction, purification was carried out by silica column chromatography (hexane/EtOAc = 100/1 → 50/1 → 20/1 (volume ratio)) to obtain tert-butyl 4-{4-[(trityl)thio]phenyl} piperazine-1-carboxylate (98.2 mg, yield: 91%) as a red powder.

¹H NMR (500 MHz, CDCl₃) δ 7.40 - 7.37 (m, 6H), 7.24 - 7.14 (m, 9H), 6.84 (d, J = 8.9 Hz, 2H), 6.53 (d, J = 8.9 Hz, 2H), 3.51 (t, J = 5.0 Hz, 4H), 3.06 (t, J = 5.0 Hz, 4H), 1.47 (s, 9H).

¹³C{¹H} NMR (101 MHz, CDCl₃) δ 154.8, 144.9, 137.5, 130.1, 127.6, 126.6, 115.6, 80.1, 70.8, 48.6, 28.5 (three carbon signals overlapped.)

### [Example 12]

N-Boc-5-[(trityl)thiol]-indole was synthesized by the production method A described in Production Example 1.

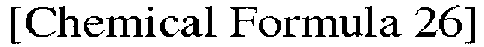

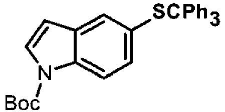

More specifically, it was prepared from N-Boc-5-bromoindole (0.2 mmol, 59.2 mg) and the catalyst 1 (5 mol%, 8.0 mg) in accordance with the production method A. After the reaction, purification was carried out by silica column chromatography (hexane/DCM = 10/1 → 5/1 (volume ratio)) to obtain N-Boc-5-[(trityl)thiol]-indole (61.1 mg, yield: 62%) as a white powder.

¹H NMR (400 MHz, CDCl₃) δ 7.78 (d, J = 8.6 Hz, 1H), 7.47 (d, J = 3.7 Hz, 1H), 7.39 (m, 6H), 7.24 - 7.11 (m, 9H), 7.05 (d, J = 1.5 Hz, 1H), 6.98 (dd, J = 8.7, 1.8 Hz, 1H), 6.30 (dd, J = 3.7, 0.6 Hz, 1H), 1.62 (s, 9H).

¹³C{¹H} NMR (101 MHz, CDCl₃) δ 144.8, 131.9, 130.7, 130.2, 130.2, 130.2, 129.2, 127.7, 127.7, 127.7, 127.3, 126.7, 126.3, 114.7, 107.3, 71.0, 28.3

### [Example 13]

4'-chloro-4-[(trityl)thio]-1,1'-biphenyl was synthesized by the production method B described in Production Example 2.

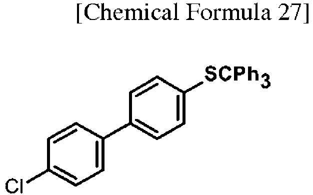

More specifically, it was prepared from 4-bromo-4'-chloro-1,1'-biphenyl (0.2 mmol, 53.5 mg) and the catalyst 2 (1 mol%, 2.2 mg) in accordance with the production method B. After the reaction, purification was carried out by silica column chromatography (hexane/DCM = 20/1 (volume ratio)) to obtain 4'-chloro-4-[(trityl)thio]-1,1'-biphenyl (91.3 mg, yield: 99%) as a white powder.

¹H NMR (400 MHz, CDCl₃) δ 7.45 - 7.32 (m, 10H), 7.27 - 7.17 (m, 11H), 7.03 - 6.98 (m, 2H).

¹³C{¹H} NMR (101 MHz, CDCl₃) δ 144.6, 139.1, 138.9, 134.8, 134.3, 133.7, 130.2, 129.0, 128.3, 127.9, 126.9, 126.6, 71.0.

### [Example 14]

2-Fluoro-4-[(trityl)thio]-1,1'-biphenyl was synthesized by the production method B described in Production Example 2.

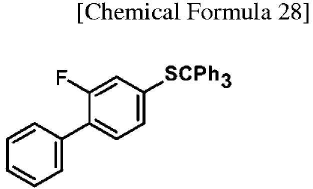

More specifically, it was prepared from 4-bromo-2-fluorobiphenyl (0.2 mmol, 50.2 mg) and the catalyst 2 (2 mol%, 4.4 mg) in accordance with the production method B. After the reaction, purification was carried out by silica column chromatography (hexane/DCM = 40/1 (volume ratio)) to obtain 2-fluoro-4-[(trityl)thio]-1,1'-biphenyl (67.1 mg, yield: 75%) as a white powder.

¹H NMR (400 MHz, CDCl₃) δ 7.45 - 7.36 (m, 10H), 7.29 - 7.16 (m, 10H), 7.08 (t, J = 8.2 Hz, 1H), 6.83 (dd, J = 8.1, 1.8 Hz, 1H), 6.69 (dd, J = 11.4, 1.8 Hz, 1H).

¹³C{¹H} NMR (101 MHz, CDCl₃) δ 158.8 (d, 1JC-F = 250.2 Hz), 144.3, 136.0 (d, J = 8.1 Hz), 135.3 (d, J = 1.4 Hz), 130.1, 130.0 (d, J = 4.0 Hz), 129.8 (d, J = 3.4 Hz), 129.0, 129.0, 128.6, 128.0, 127.9, 127.1, 121.24 (d, J = 24.4 Hz), 71.3.

¹⁹F NMR (376 MHz, CDCl₃) δ -117.83.

### [Example 15]

2-{4-[(trityl)thio]phenyl}benzoxazole was synthesized by the production method B described in Production Example 2.

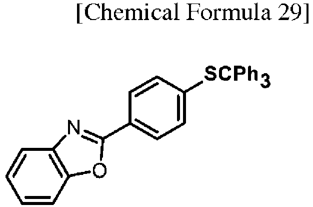

More specifically, it was prepared from 2-(4-bromophenyl)benzoxazole (0.2 mmol, 54.8 mg) and the catalyst 2 (2 mol%, 4.4 mg) in accordance with the production method B. After the reaction, purification was carried out by silica column chromatography (hexane/DCM = 20/1 (volume ratio)) to obtain 2-{4-[(trityl)thio]phenyl}benzoxazole (79.8 mg, yield: 85%) as a white powder.

¹H NMR (400 MHz, CDCl₃) δ 7.86 (d, J = 8.4 Hz, 2H), 7.76 - 7.67 (m, 1H), 7.56 - 7.47 (m, 1H), 7.42 (d, J = 8.1 Hz, 6H), 7.36 - 7.18 (m, 11H), 7.07 (d, J = 8.4 Hz, 2H).

¹³C{¹H} NMR (101 MHz, CDCl₃) δ 162.8, 150.8, 144.2, 142.2, 140.4, 132.8, 130.1, 128.1, 128.0, 127.2, 125.4, 125.3, 124.7, 120.1, 110.7, 71.3.

### [Example 16]

2-Methyl-5-[(trityl)thio]pyrimidine was synthesized by the production method B described in Production Example 2.

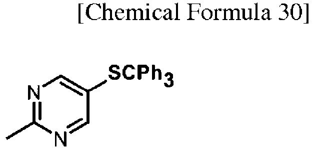

More specifically, it was prepared from 5-bromo-2-methylpyrimidine (0.2 mmol, 34.6 mg) and the catalyst 2 (5 mol%, 11.1 mg) in accordance with the production method B. After the reaction, purification was carried out by silica column chromatography (hexane/TEA = 100/1 (volume ratio)) to obtain 2-methyl-5-[(trityl)thio]pyrimidine (64.9 mg, yield: 88%) as a white powder.

¹H NMR (400 MHz, CDCl₃) δ 8.06 (s, 2H), 7.44 - 7.39 (m, 6H), 7.29 - 7.19 (m, 9H), 2.59 (s, 2H).

¹³C{¹H} NMR (101 MHz, CDCl₃) δ 167.2, 161.9, 143.7, 129.7, 128.0, 127.2, 126.5, 71.7, 25.6.

### [Example 17]

2-Phenyl-5-[(trityl)thio]-pyridine was synthesized by the production method B described in Production Example 2.

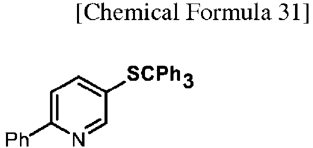

More specifically, it was prepared from 5-bromo-2-phenylpyrimidine (0.2 mmol, 46.8 mg) and the catalyst 2 (5 mol%, 11.1 mg) in accordance with the production method B. After the reaction, purification was carried out by silica column chromatography (hexane/DCM = 1/1 (volume ratio)) to obtain 2-phenyl-5-[(trityl)thio]-pyridine (74.9 mg, yield: 87%) as a white powder.

¹H NMR (400 MHz, CDCl₃) δ 8.32 (d, J = 2.1 Hz, 1H), 7.87 (dd, J = 8.0, 1.3 Hz, 2H), 7.48 - 7.42 (m, 8H), 7.37 - 7.12 (m, 12H).

¹³C{¹H} NMR (101 MHz, CDCl₃) δ 147.0, 144.3, 142.8, 130.0, 129.3, 128.9, 128.1, 128.1, 128.0, 127.4, 127.1, 127.0, 119.8, 71.6.

### [Example 18]

4-Cyanophenyltrityl sulfide was synthesized by the production method B described in Production Example 2.

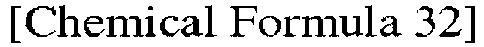

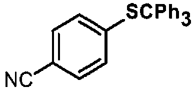

More specifically, it was prepared from 4-bromobenzonitrile (0.05 mmol, 9.1 mg) and the catalyst 2 (5 mol%, 2.8 mg) in accordance with the production method B. After the reaction, purification was carried out by silica column chromatography (hexane/EtOAc = 10/1 (volume ratio)) to obtain 4-cyanophenyltrityl sulfide (14.1 mg, yield: 75%) as a white powder.

¹H NMR (400 MHz, CDCl₃) δ 7.39 - 7.35 (m, 6H), 7.30 - 7.19 (m, 11H), 6.99 (d, J = 8.3 Hz, 2H).

¹³C{¹H} NMR (101 MHz, CDCl₃) δ 143.7, 143.4, 131.5, 131.3, 130.0, 128.2, 127.4, 118.9, 109.5, 71.5.

### [Example 19]

Ethyl 5-[(trityl)thio)-2-furancarboxylate was synthesized by the production method B described in Production Example 2.

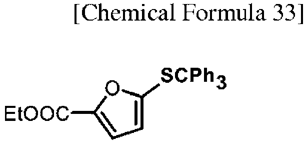

More specifically, it was prepared from ethyl 5-bromo-2-furancarboxylate (0.05 mmol, 11.0 mg, 7.2 µL) and the catalyst 2 (5 mol%, 2.8 mg) in accordance with the production method B. After the reaction, purification was carried out by silica column chromatography (hexane/Et2O = 20/1 (volume ratio)) to obtain ethyl 5-[(trityl)thio)-2-furancarboxylate (10.6 mg, yield: 51%) as a white powder.

¹H NMR (400 MHz, CDCl₃) δ 7.38 - 7.34 (m, 6H), 7.28 - 7.17 (m, 9H), 6.83 (d, J = 3.5 Hz, 1H), 5.97 (d, J = 3.5 Hz, 1H), 4.27 (q, J = 7.1 Hz, 2H), 1.32 (t, J = 7.1 Hz, 3H).

¹³C{¹H} NMR (101 MHz, CDCl₃) δ 158.2, 149.9, 146.9, 144.1, 130.0, 127.9, 127.2, 121.9, 118.7, 73.4, 61.1, 14.4.

### [Example 20]

4-Nitrophenyltrityl sulfide was synthesized by the production method B described in Production Example 2.

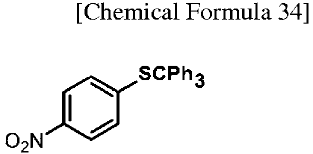

More specifically, it was prepared from 4-bromonitrobenzene (0.05 mmol, 10.1 mg) and the catalyst 2 (5 mol%, 2.8 mg) in accordance with the production method B. After the reaction, purification was carried out by silica column chromatography (hexane/Et2O = 20/1 (volume ratio)) to obtain 4-nitrophenyltrityl sulfide (11.9 mg, yield: 60%) as a white powder.

¹H NMR (400 MHz, CDCl₃) δ 7.85 - 7.79 (m, 2H), 7.40 - 7.35 (m, 6H), 7.31 - 7.20 (m, 9H), 7.06 - 7.00 (m, 2H).

¹³C{¹H} NMR (101 MHz, CDCl₃) δ 146.44, 145.67, 143.47, 130.37, 130.03, 128.22, 127.53, 123.12, 71.53.

### [Example 21]

Methyl 3,5-bis[(trityl)thio]benzoate was synthesized by the production method B described in Production Example 2.

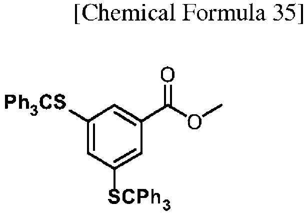

More specifically, it was prepared from 3,5-dibromobenzene (0.05 mmol, 14.7 mg) and the catalyst 2 (10 mol%, 5.6 mg) in accordance with the production method B. After the reaction, purification was carried out by silica column chromatography (hexane/EtOAc = 10/1 (volume ratio)) to obtain methyl 3,5-bis[(trityl)thio]benzoate (28.2 mg, yield: 82%) as a white powder.

¹H NMR (400 MHz, CDCl₃) δ 7.33 - 7.28 (m, 12H), 7.22 (d, J = 1.7 Hz, 2H), 7.21 - 7.12 (m, 18H), 6.98 (t, J = 1.8 Hz, 1H), 3.68 (s, 3H).

¹³C{¹H} NMR (101 MHz, CDCl₃) δ 165.7, 144.2, 143.9, 134.9, 134.9, 130.0, 129.6, 128.1, 128.1, 127.9, 126.9, 71.5, 52.0.

### [Example 22]

3,5-bis[(trityl)thio]chlorobenzene was synthesized by the production method B described in Production Example 2.

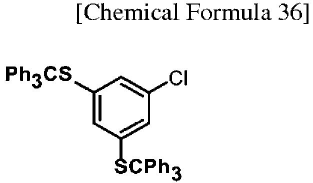

More specifically, it was prepared from 1,3-dibromo-5-chlorobenzene (0.05 mmol, 13.5 mg) and the catalyst 2 (10 mol%, 5.6 mg) in accordance with the production method B. After the reaction, purification was carried out by silica column chromatography (hexane/EtOAc = 20/1 (volume ratio)) to obtain 3,5-bis[(trityl)thio]chlorobenzene (32.0 mg, yield: 97%) as a white powder.

¹H NMR (400 MHz, CDCl₃) δ 7.32 - 7.28 (m, 12H), 7.22 - 7.17 (m, 18H), 6.84 (t, J = 1.3 Hz, 1H), 6.42 (dd, J = 1.6, 0.5 Hz, 2H).

¹³C{¹H} NMR (101 MHz, CDCl₃) δ 144.1, 135.7, 130.0, 129.4, 127.9, 127.5, 127.2, 127.0, 71.6.

### [Example 23]

4,4'-di[(trityl)thio]-trans-stilbene was synthesized by the production method B described in Production Example 2.

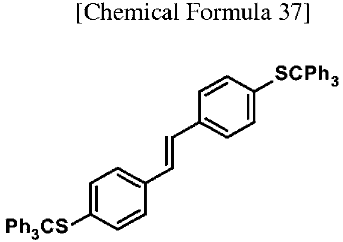

More specifically, it was prepared from 4,4'-dibromo-trans-stilbene (0.05 mmol, 16.9 mg) and the catalyst 2 (10 mol%, 5.6 mg) in accordance with the production method B. After the reaction, purification was carried out by silica column chromatography (hexane/DCM = 10/3 (volume ratio)) to obtain 4,4'-di[(trityl)thio]-trans-stilbene (30.1 mg, yield: 82%) as a white powder.

¹H NMR (400 MHz, CDCl₃) δ 7.43 - 7.37 (m, 12H), 7.26 - 7.15 (m, 18H), 7.08 (d, J = 8.5 Hz, 4H), 6.91 (d, J = 8.5 Hz, 4H), 6.83 (s, 2H).

¹³C{¹H} NMR (101 MHz, CDCl₃) δ 144.6, 136.4, 134.6, 134.1, 130.1, 128.3, 127.8, 126.8, 126.2, 71.0.

### [Example 24]

6-[(trityl)thio]quinoxaline was synthesized by the production method B described in Production Example 2.

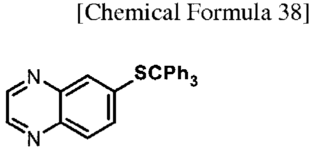

More specifically, it was prepared from 6-bromoquinoxaline (0.05 mmol, 10.5 mg) and the catalyst 2 (5 mol%, 2.8 mg) in accordance with the production method B. After the reaction, purification was carried out by silica column chromatography (hexane/EtOAc = 1/1 (volume ratio)) to obtain 6-[(trityl)thio]quinoxaline (17.4 mg, yield: 86%) as a white powder.

¹H NMR (400 MHz, CDCl₃) δ 8.69 (q, J = 1.9 Hz, 2H), 7.72 (d, J = 8.9 Hz, 1H), 7.62 (d, J = 2.1 Hz, 1H), 7.46 (m, 6H), 7.35 (dd, J = 8.8, 2.1 Hz, 1H), 7.29 - 7.17 (m, 9H).

¹³C{¹H} NMR (101 MHz, CDCl₃) δ 145.2, 144.5, 143.8, 142.6, 141.8, 139.4, 133.5, 131.6, 130.1, 128.5, 128.1, 127.2, 71.5.

### [Example 25]

4-Methoxyphenyltrityl sulfide was synthesized by the production method C described in Production Example 3.

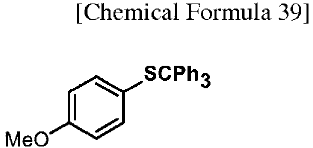

More specifically, it was prepared from 4-chloromethoxybenzene and the catalyst 1 or catalyst 2 in accordance with the production method C. The yield was 4% when the catalyst 1 was used, and the yield was 16% when the catalyst 2 was used.

### [Example 26]

4-Nitrophenyltrityl sulfide was synthesized by the production method C described in Production Example 3.

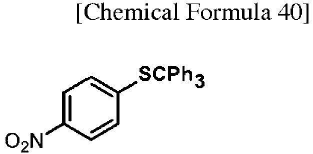

More specifically, it was prepared from 4-chloronitrobenzene and the catalyst 1 or catalyst 2 in accordance with the production method C. The yield was 8% when the catalyst 1 was used, and the yield was 13% when the catalyst 2 was used.

### [Production Example 4] Typical production method D for synthesizing aryl trityl sulfide compounds

A test tube dried in an oven was moved into a glove box under an argon atmosphere. After adding aryl iodide to a mixture of the catalyst 1 (5 mol%) and toluene (0.5 M) in this test tube, the test tube was cooled in a refrigerator at -30°C for 0.5 hours. KSTr (1.5 equivalents) was added to this mixture with vigorous stirring at room temperature. After sealing with an aluminum cap with a septum using a vial crimper, the test tube was taken out of the glove box, and the resulting mixture was stirred at room temperature for 12 hours. The obtained reaction mixture was diluted with CDCl₃, and 1,4-bis(trifluoromethyl)benzene (15.5 µL, 0.1 mmol) was added as an internal standard to determine the NMR yield of the aryl trityl sulfide compound.

### [Example 27]

4-Methoxyphenyltrityl sulfide was synthesized by the production method D described in Production Example 4.

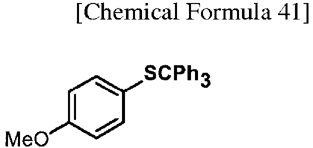

More specifically, it was prepared from 4-iodoanisole and the catalyst 1 in accordance with the production method D. The yield was 95%.

### [Example 28]

2-Methoxy-5-[(trityl)thio]-pyridine was synthesized by the production method D described in Production Example 4.

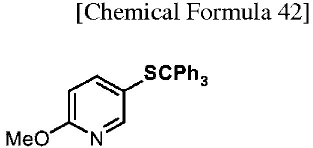

More specifically, it was prepared from 5-iodo-2-methoxypyridine and the catalyst 1 in accordance with the production method D. The yield was 74%.

### [Example 29]

6-[(trityl)thio]quinoline was synthesized by the production method D described in Production Example 4.

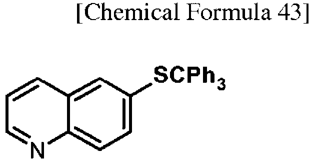

More specifically, it was prepared from 6-iodo-quinoline and the catalyst 1 in accordance with the production method D. The yield was 53%.

### [Example 30]

Methyl 4-(trityl)thiobenzoate was synthesized by the production method D described in Production Example 4.

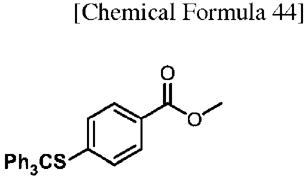

More specifically, it was prepared from methyl 4-iodobenzoate and the catalyst 1 in accordance with the production method D. The yield was 82%.

### [Production Example 5] Typical production method E for synthesizing aryl trityl sulfide compounds

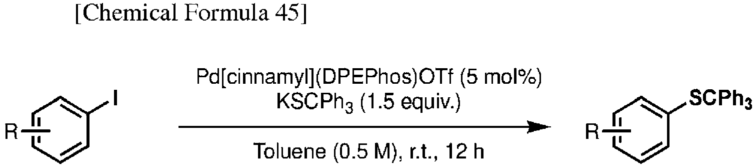

A test tube dried in an oven was moved into a glove box under an argon atmosphere. After adding aryl iodide (0.2 mmol) to a mixture of the catalyst 3 (9.1 mg, 5 mol%) and toluene (0.4 mL, 0.5 M) in this test tube, KSTr (KSCPh₃) (94.3 mg, 0.3 mmol, 1.5 equivalents) was added to this mixture with vigorous stirring at room temperature. After sealing with an aluminum cap with a septum using a vial crimper, the test tube was taken out of the glove box, and the resulting mixture was stirred at room temperature for 12 hours. The solvent of the reaction mixture was removed by a rotary evaporator, and the crude material was purified by silica column chromatography to obtain the corresponding aryl trityl sulfide.

### [Example 31]

4-[(trityl)thio]benzophenone was synthesized by the production method E described in Production Example 5.

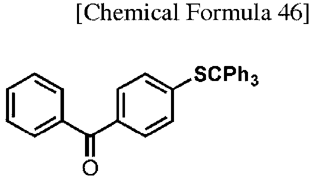

More specifically, it was prepared from 4-iodobenzophenone (0.2 mmol, 61.6 mg) and the catalyst 3 (5 mol%, 9.1 mg) in accordance with the production method E. After the reaction, 4-[(trityl)thio]benzophenone (77.6 mg, yield: 85%) was obtained as a white powder through purification by silica column chromatography (hexane/ethyl acetate = 10:1 (volume ratio)).

¹H NMR (400 MHz, CDCl₃) δ 7.71 - 7.66 (m, 2H), 7.59 - 7.52 (m, 1H), 7.47 - 7.38 (m, 10H), 7.30 - 7.18 (m, 9H), 7.06 - 7.00 (m, 2H).

¹³C NMR (101 MHz, CDCl₃) δ 196.2, 144.1, 142.2, 137.7, 135.2, 132.5, 131.2, 130.1, 130.0, 129.8, 128.4, 128.0, 127.2, 71.2.

### [Example 32]

4-Bromophenyltrityl sulfide was synthesized by the production method E described in Production Example 5.

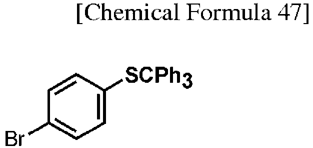

More specifically, it was prepared from 1-bromo-4-iodobenzene (0.6 mmol, 169.8 mg) and the catalyst 3 (5 mol%, 27.3 mg) in accordance with the production method E. After the reaction, 4-bromophenyltrityl sulfide (221.1 mg, yield: 85%) was obtained as a white powder through purification by silica column chromatography (hexane/methylene chloride = 10:1 (volume ratio)).

¹H NMR (400 MHz, CDCl₃) δ 7.39 (dd, J = 8.2, 1.3 Hz, 6H), 7.34 - 7.17 (m, 9H), 7.12 (d, J = 8.5 Hz, 2H), 6.79 (d, J = 8.5 Hz, 2H).

### [Example 33]

4'-Bromo-4-[(trityl)thio]-1,1'-biphenyl was synthesized by the production method E described in Production Example 5.

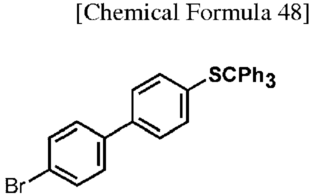

More specifically, it was prepared from 4-bromo-4'-iodobiphenyl (0.5 mmol, 179.5 mg) and the catalyst 3 (5 mol%, 22.8 mg) in accordance with the production method E. After the reaction, 4'-bromo-4-[(trityl)thio]-1,1'-biphenyl (231.0 mg, yield: 91%) was obtained as a white powder through purification by silica column chromatography (hexane/methylene chloride = 10:1 (volume ratio)).

¹H NMR (400 MHz, CDCl₃) δ 7.55 - 7.47 (m, 2H), 7.45 - 7.39 (m, 6H), 7.36 - 7.31 (m, 2H), 7.31 - 7.12 (m, 11H), 7.05 - 6.95 (m, 2H).

### [Example 34]

4-[(trityl)thio]-benzonitrile was synthesized by the production method E described in Production Example 5.

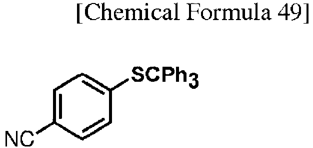

More specifically, it was prepared from 4-iodobenzonitrile (0.5 mmol, 179.5 mg) and the catalyst 3 (5 mol%, 22.8 mg) in accordance with the production method E. After the reaction, 4-[(trityl)thio]-benzonitrile (173.7 mg, yield: 92%) was obtained as a white powder through purification by silica column chromatography (hexane/ethyl acetate = 10:1 (volume ratio)).

¹H NMR (400 MHz, CDCl₃) δ 7.39 - 7.35 (m, 6H), 7.30 - 7.19 (m, 11H), 6.99 (d, J = 8.3 Hz, 2H).

¹³C NMR (101 MHz, CDCl₃) δ 143.7, 143.4, 131.5, 131.3, 130.0, 128.2, 127.4, 118.9, 109.5, 71.5.

### [Example 35]

5-[(trityl)thio]-2,2-difluoro-1,3-benzodioxole was synthesized by the production method B described in Production Example 2.

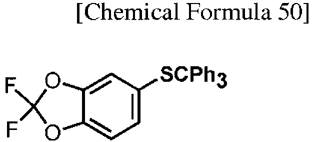

More specifically, it was prepared from 5-bromo-2,2-difluoro-1,3-benzodioxole (0.1 mmol, 23.7 mg) and the catalyst 2 (5 mol%, 11.1 mg) in accordance with the production method B. After the reaction, 5-[(trityl)thio]-2,2-difluoro-1,3-benzodioxole (22.1 mg, yield: 51%) was obtained as a white powder through purification by silica column chromatography (hexane/methylene chloride = 10:3 (volume ratio)).

¹H NMR (400 MHz, CDCl₃) δ 7.43 - 7.36 (m, 6H), 7.28 - 7.17 (m, 9H), 6.76 (dd, J = 8.3, 1.7 Hz, 1H), 6.70 (d, J = 8.3 Hz, 1H), 6.58 (d, J = 1.5 Hz, 1H).

¹⁹F NMR (376 MHz, CDCl₃) δ -49.9 (s, 2F).

### [Example 36]

(3-{4-[(trityl)thio]-phenyl}-3-pyridin-2-yl-propyl)-dimethyl-amine was synthesized by the production method A described in Production Example 1.

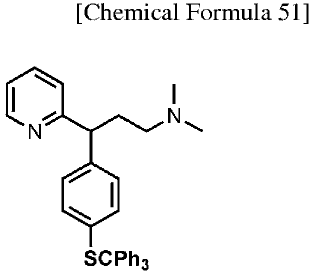

More specifically, it was prepared from brompheniramine (0.2 mmol, 63.9 mg) and the catalyst 1 (10 mol%, 15.9 mg) in accordance with the production method A. After the reaction, (3-{4-[(trityl)thio]-phenyl}-3-pyridin-2-yl-propyl)-dimethyl-amine (75.2 mg, yield: 73%) was obtained as a white powder through purification by silica column chromatography (methylene chloride).

¹H NMR (400 MHz, CDCl₃) δ8.52 (ddd, J = 4.9, 1.9, 0.9 Hz, 1H), 7.53 (td, J = 7.7, 1.8 Hz, 1H), 7.44 - 7.34 (m, 6H), 7.25 - 7.10 (m, 9H), 7.08 (ddd, J = 7.5, 4.9, 1.2 Hz, 1H), 7.02 (dt, J = 7.9, 1.1 Hz, 1H), 6.97 - 6.92 (m, 2H), 6.90 - 6.85 (m, 2H), 3.98 (t, J = 7.5 Hz, 1H), 2.39 - 2.23 (m, 1H), 2.18 (s, 6H), 2.16 - 2.04 (m, 3H).

### [Example 37]

2',3',5'-tri-O-acetyl-6-chloro-2-[(trityl)thio]purine riboside was synthesized by the production method E described in Production Example 5.

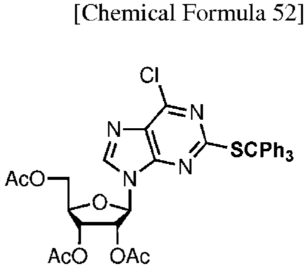

More specifically, it was prepared from 2',3',5'-tri-O-acetyl-6-chloro-2-iodopurine riboside (0.2 mmol, 107.7 mg) and the catalyst 3 (5 mol%, 9.1 mg) in accordance with the production method E. After the reaction, (2',3',5'-tri-O-acetyl-6-chloro-2-[(trityl)thio]purine riboside (121.4 mg, yield: 89%) was obtained as a white powder through purification by silica column chromatography (hexane/ethyl acetate = 2:1 (volume ratio)).

¹H NMR (400 MHz, CDCl₃) δ7.95 (s, 1H), 7.46 - 7.36 (m, 6H), 7.33 - 7.16 (m, 9H), 6.09 (d, J = 5.1 Hz, 1H), 5.73 (t, J = 5.3 Hz, 1H), 5.62 - 5.55 (m, 1H), 4.45 - 4.27 (m, 3H), 2.14 (s, 3H), 2.10 (s, 3H), 2.09 (s, 3H).

¹³C NMR (101 MHz, CDCl₃) δ170.4, 169.7, 169.5, 161.4, 148.6, 143.4, 140.5, 131.8, 130.3, 127.7, 127.1, 117.5, 86.4, 80.5, 73.4, 70.5, 21.0, 20.7, 20.6.

### [Example 38]

4-[(trityl)thio]-N-methoxy-N-methylbenzamide was synthesized by the production method E described in Production Example 5.

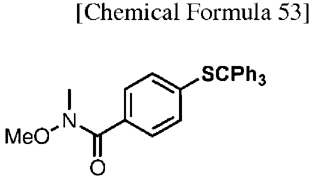

More specifically, it was prepared from 4-iodo-N-methoxy-N-methylbenzamide (1.0 mmol, 291.1 mg) and the catalyst 3 (5 mol%, 45.6 mg) in accordance with the production method E. After the reaction, 4-[(trityl)thio]-N-methoxy-N-methylbenzamide (357.6 mg, yield: 81%) was obtained as a white powder through purification by silica column chromatography (hexane/ethyl acetate = 5:2 (volume ratio)).

¹H NMR (400 MHz, CDCl₃) δ7.43 - 7.37 (m, 6H), 7.31 (d, J = 7.9 Hz, 2H), 7.27 - 7.17 (m, 9H), 7.01 - 6.92 (m, 2H), 3.46 (s, 3H), 3.28 (s, 3H).

4-Iodo-N-methoxy-N-methylbenzamide was produced as follows.

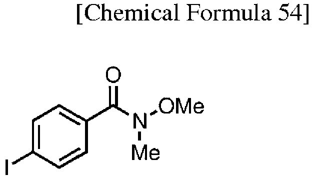

In a 50 mL round bottom flask in a glove box under an argon atmosphere, N,O-dimethylhydroxylamine hydrochloride (1.07 g, 11 mmol, 1.1 equivalents) was added to a stirred suspension of 4-iodobenzoyl chloride (2.67 g, 10 mmol) and DCM (15 mL). This flask was sealed with a septum, and then taken out of the glove box and cooled in an ice bath. A solution of pyrimidine (1.8 mL, 22 mmol, 2.2 equivalents) dissolved in 5 mL of DCM was added to this stirred suspension at 0°C. The reaction mixture was heated to room temperature and stirred for 12 hours. Subsequently, the obtained reaction solution was washed once with water and then twice with a saturated sodium bicarbonate solution. The organic layers obtained by washing were combined and dried over anhydrous sodium sulfate, and the solvent was removed using a rotary evaporator to obtain 4-iodo-N-methoxy-N-methylbenzamide (2.05 g, yield: 70%) as a colorless oil.

¹H NMR (400 MHz, CDCl₃) δ 7.76 (d, J = 6.7 Hz, 2H), 7.43 (d, J = 6.7 Hz, 2H), 3.54 (s, 3H), 3.35 (s, 3H).

¹³C NMR (101 MHz, CDCl₃) δ 169.0, 137.3, 133.5, 130.1, 97.5, 61.3, 33.6.

### [Production Example 6] Typical production method F for synthesizing SF₅-containing aryl compounds

Using an aryl trityl sulfide compound as a substrate, an SF₅-containing aryl compound can be synthesized by the following method.

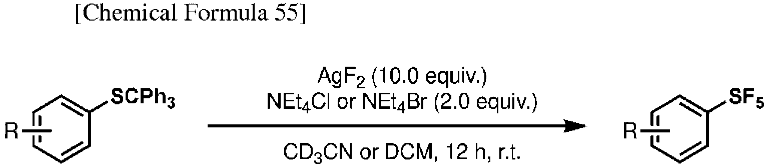

In a glove box under an argon atmosphere, NEt₄Cl or NEt₄Br (0.2 mmol, 2.0 equivalents), an aryl trityl sulfide compound (0.1 mmol, 1.0 equivalents), and a magnetic stirrer bar were placed in a glass vial. MeCN or DCM (0.5 mL) was added and the reaction mixture was stirred for 2 minutes. Subsequently, AgF₂ (1.0 mmol, 10.0 equivalents) was added all at once to a solution vigorously stirred at room temperature, and the vial was closed with a screw cap. Within about 1 minute, the initially black suspension turned orange and the organic layer became reddish purple. The reaction solution was stirred for 12 hours. The NMR yield was determined by adding 1,4-bis(trifluoromethyl)benzene (15.5 µL, 0.1 mmol) to the reaction mixture as an internal standard.

The NMR yield was determined by adding 1,4-bis(trifluoromethyl)benzene (BTB, 1.0 equivalent with respect to the SF₅ product) to the reaction mixture as an internal standard and filtering this reaction mixture.

The resulting mixture was injected into an NMR tube through a PTFE syringe filter, and the ¹⁹F NMR signals of the SF₅ and CF₃ groups, respectively, were integrated. After the NMR measurement, the solvent was removed by rotary evaporation, and the crude product was purified by silica column chromatography to obtain the corresponding SF₅-containing aryl compound.

### [Example 39]

5'-(pentafluorosulfanyl)-m-terphenyl was synthesized by the production method F described in Production Example 6.

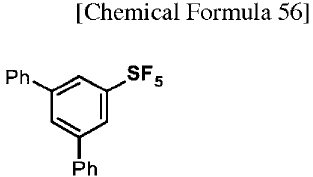

More specifically, it was synthesized from 5'-tritylthiol-m-terphenyl using NEt₄Br in DCM in accordance with the production method F (NMR yield: 75%). 5'-(pentafluorosulfanyl)-m-terphenyl (24.8 mg, yield: 70%) was obtained as a white powder through purification by silica column chromatography using hexane as an eluent.

¹H NMR (400 MHz, CDCl₃) δ 7.92 (m, 3H), 7.65 - 7.59 (m, 4H), 7.53 - 7.47 (m, 4H), 7.45 - 7.40 (m, 2H).

¹³C NMR (100 MHz, CDCl₃) δ 155.5-154.7 (m), 142.9, 139.6, 129.3, 129.2, 128.5, 127.5, 123.6 (quint, 3JCF = 4.5 Hz).

¹⁹F NMR (375 MHz, CDCl₃) δ 84. (quint, 2JFF, ax = 149.3 Hz, 1F), 63.1 (d, 2JFF, eq = 149.3 Hz, 4F).

### [Example 40]

2-(pentafluorosulfanyl)dibenzofuran was synthesized by the production method F described in Production Example 6.

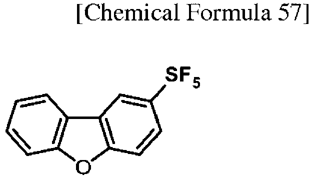

More specifically, it was synthesized from 2-(tritylthio)-dibenzofuran using NEt₄Br in MeCN in accordance with the production method F (NMR yield: 85%). 2-(pentafluorosulfanyl)dibenzofuran (21.5 mg, yield: 73%) was obtained as a white powder through purification by silica column chromatography using hexane as an eluent.

¹H NMR (400 MHz, CDCl₃) δ 8.36 (d, J = 2.3 Hz, 1H), 8.02 - 7.96 (m, 1H), 7.87 (dd, J = 9.0, 2.3 Hz, 1H), 7.61 (m, 2H), 7.57-7.51 (m, 1H), 7.41 (td, J = 7.8, 1.0 Hz, 1H).

¹³C NMR (100 MHz, CDCl₃) δ 157.2, 156.7, 149.2 (quint, 2JCF = 18.2 Hz), 128.6, 125.0 (quint, 3JCF = 4.7 Hz), 124.4, 123.7, 123.4, 121.1, 119.3 (quint, 3JC-F = 4.7 Hz), 112.2, 111.6.

¹⁹F NMR (375 MHz, CDCl₃) δ 85.7 (quint, 2JFF, ax = 150.7 Hz, 1F), 65.3 (d, 2JFF, eq = 150.7 Hz, 4F).

### [Example 41]

4-(pentafluorosulfanyl)-4'-chloro-1,1'-biphenyl was synthesized by the production method F described in Production Example 6.

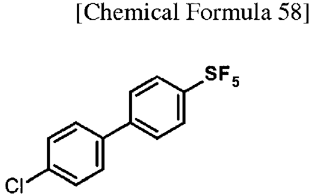

More specifically, it was synthesized from 4'-chloro-1,1'-biphenyl-4-trityl sulfide using NEt₄Br in MeCN in accordance with the production method F (NMR yield: 95%). 4-(pentafluorosulfanyl)-4'-chloro-1,1'-biphenyl (29.8 mg, yield: 95%) was obtained as a white powder through purification by silica column chromatography using hexane as an eluent.

¹H NMR (400 MHz, CDCl₃) δ 7.85 - 7.79 (m, 2H), 7.64 - 7.59 (m, 2H), 7.54 - 7.48 (m, 2H), 7.47 - 7.41 (m, 2H).

¹³C NMR (100 MHz, CDCl₃) δ 153.2 (quint, 2JCF = 18.2 Hz), 143.4, 137.6, 134.9, 129.4, 128.7, 127.3, 126.7 (quint, 3JCF = 4.6 Hz).

¹⁹F NMR (375 MHz, CDCl₃) δ 84.6 (quint, 2JFF, ax = 150.5 Hz, 1F) 63.2 (d, 2JFF, eq = 150.5 Hz, 4F).

### [Example 42]

1-(pentafluorosulfanyl)-4-(trans-4-propylcyclohexyl)benzene was synthesized by the production method F described in Production Example 6.

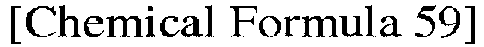

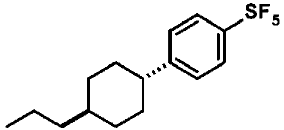

More specifically, it was synthesized from 1-[4-(trans-4'-propylcyclohexyl)phenyl]trityl sulfide using NEt₄Br in MeCN in accordance with the production method F (NMR yield: 86%). 1-(pentafluorosulfanyl)-4-(trans-4-propylcyclohexyl)benzene (23.6 mg, yield: 72%) was obtained as a white powder through purification by silica column chromatography using hexane as an eluent.

¹H NMR (400 MHz, CDCl₃) δ 7.75 - 7.53 (m, 2H), 7.27 (d, J = 8.3 Hz, 2H), 2.59 - 2.41 (m, 1H), 1.88 (dd, J = 10.9, 1.7 Hz, 4H), 1.50 - 1.18 (m, 7H), 1.11 - 0.98 (m, 2H), 0.90 (t, J = 7.2 Hz, 3H).

¹³C NMR (100 MHz, CDCl₃) δ 151.9, 151.9 (quint, 2JCF = 18.2 Hz) 127.2, 126.0 (quint, 3JCF = 5.0 Hz), 44.4, 39.7, 37.0, 34.2, 33.5, 20.1, 14.5.

¹⁹F NMR (375 MHz, CDCl₃) δ 85.6 (quint, 2JFF, ax = 149.1 Hz, 1F), 63.3 (d, 2JFF, eq = 149.1 Hz, 4F).

### [Example 43]

4-(pentafluorosulfanyl)-2-fluorobiphenyl was synthesized by the production method F described in Production Example 6.

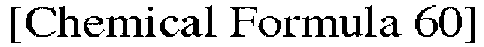

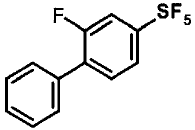

More specifically, it was synthesized from 4-(tritylthio)-2-fluorobiphenyl using NEt₄Br in MeCN in accordance with the production method F (NMR yield: 98%). 4-(pentafluorosulfanyl)-2-fluorobiphenyl (32.4 mg, yield: 94%) was obtained as a white powder through purification by silica column chromatography using hexane as an eluent.

¹H NMR (400 MHz, CDCl₃) δ 7.61 (ddd, J = 12.6, 9.5, 2.2 Hz, 2H), 7.57 - 7.51 (m, 3H), 7.51-7.41 (m, 3H).

¹³C NMR (100 MHz, CDCl₃) δ. 158.6 (d, 1JC-F = 251.7 Hz), 153.2 (d-quint, 2JC-F = 18.7, 3JCF = 8.1 Hz), 133.9 (d, J = 1.5 Hz), 132.9 (d, 2JC-F = 13.5 Hz), 130.8 (d, 3JC-F = 3.7 Hz), 129.1 (d, J = 3.2 Hz), 128.9 (d, J = 3.2 Hz), 122.3 - 121.9 (m), 115.0 (d-quint, 2JC-F = 28.5, 3JC-F = 4.9 Hz).

¹⁹F NMR (375 MHz, CDCl₃) δ 83.3 (quint, 2JFF, ax = 150.5 Hz), 63.2 (d, 2JFF, eq = 150.5 Hz), -114.2 (t, J = 7.5 Hz).

### [Example 44]

3,5-Dimethoxyphenyl-pentafluorosulfide was synthesized by the production method F described in Production Example 6.

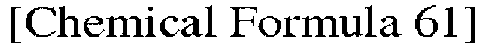

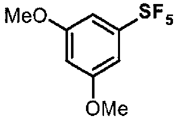

More specifically, it was synthesized from 3,5-dimethoxyphenyltrityl sulfide using NEt₄Br in MeCN in accordance with the production method F (NMR yield: 39%).

¹H NMR (400 MHz, CDCl₃) δ 6.91 (d, J = 2.1 Hz, 2H), 6.68 (s, 2H), 3.78 (s, 6H).

¹⁹F NMR (375 MHz, CDCl₃) δ 84.5 (quint, 2JFF, ax = 148.5 Hz, 1F), 62.3 (d, 2JFF, eq = 148.5 Hz, 4F).

### [Example 45]

3,4,5-trimethoxyphenyl-pentafluorosulfide was synthesized by the production method F described in Production Example 6.

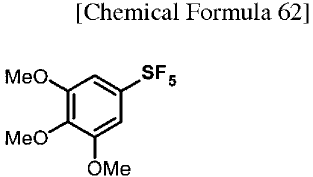

More specifically, it was synthesized from 3,4,5-trimethoxyphenyltrityl sulfide using NEt₄Br in MeCN in accordance with the production method F (NMR yield: 7%).

¹H NMR (only composite signals were detected).

¹⁹F NMR (375 MHz, CDCl₃) δ 81.1 (quint, 2JFF, ax = 148.8 Hz, 1F), 57.3 (d, 2JFF, eq = 148.8 Hz, 4F).

### [Example 46]

4-Bromophenyl-pentafluorosulfide was synthesized by the production method F described in Production Example 6.

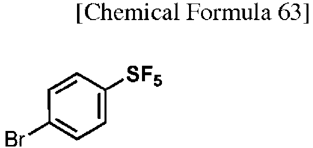

More specifically, it was synthesized from 4-bromophenyltrityl sulfide using NEt₄Cl in MeCN in accordance with the production method F (NMR yield: 87%). 4-bromophenyl-pentafluorosulfide (21.2 mg, yield: 75%) was obtained as a white powder through purification by silica column chromatography using hexane as an eluent.

¹H NMR (400 MHz, CDCl₃) δ 7.68 - 7.54 (m, 4H).

¹³C NMR (100 MHz, CDCl₃) δ 153.2 - 152.4 (m), 132.1, 127.7 (quint, ³J_{CF} = 4.7 Hz), 126.3 (quint, ⁴J_{CF} = 1.3 Hz).

¹⁹F NMR (375 MHz, CDCl₃) δ 83.6 (quint, ²J_{FF, ax} = 151.2 Hz, 1F), 63.1 (d, ²J_{FF, eq} = 152 Hz, 4F).

### [Example 47]

4'-Bromo-4-(pentafluorosulfanyl)-1,1'-biphenyl was synthesized by the production method F described in Production Example 6.

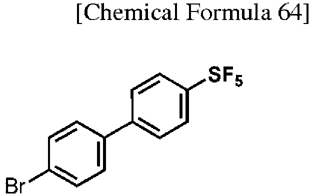

More specifically, it was synthesized from 4'-bromo-4-[(trityl)thio]-1,1'-biphenyl using NEt₄Cl in MeCN in accordance with the production method F (NMR yield: 86%). 4'-bromo-4-(pentafluorosulfanyl)-1,1'-biphenyl (27.6 mg, yield: 77%) was obtained as a white powder through purification by silica column chromatography using hexane as an eluent.

¹H NMR (400 MHz, CDCl₃) δ 7.87 - 7.79 (m, 2H), 7.66 - 7.57 (m, 4H), 7.48 - 7.41 (m, 2H).

¹³C NMR (100 MHz, CDCl₃) δ 153.2 (quint, ²J_{CF} = 17.3 Hz), 143.4, 138.1, 132.4, 129.0, 127.2, 126.7 (quint, ³J_{CF} = 4.6 Hz), 123.1.

¹⁹F NMR (375 MHz, CDCl₃) δ 84.6 (quint, ²J_{FF, ax} = 150.4 Hz, 1F), 63.2 (d, ²J_{FF, eq} = 150.1 Hz, 4F).

### [Example 48]

4-(pentafluorosulfanyl)-benzonitrile was synthesized by the production method F described in Production Example 6.

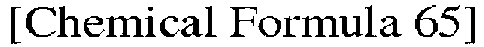

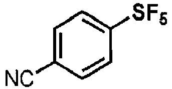

More specifically, it was synthesized from 4-[(trityl)thio]-benzonitrile using NEt₄Cl in MeCN in accordance with the production method F (NMR yield: 91%). 4-(pentafluorosulfanyl)-benzonitrile (19.0 mg, yield: 83%) was obtained as a white powder through purification by silica column chromatography using hexane as an eluent.

¹H NMR (400 MHz, CDCl₃) δ 7.90 (dd, J = 8.7, 1.3 Hz, 2H), 7.80 (d, J = 8.9 Hz, 2H).

¹³C NMR (100 MHz, CDCl₃) δ 156.6 (quint, ²J_{CF} = 19.1 Hz), 132.9, 127.2 (quint, ³J_{CF} = 4.8 Hz), 117.1, 116.0.

¹⁹F NMR (375 MHz, CDCl₃) δ 81.5 (quint, ²J_{FF, ax} = 150.7 Hz, 1F), 62.2 (d, ²J_{FF, eq} = 150.7 Hz, 4F).

### [Example 49]

4-(pentafluorosulfanyl)-N-methoxy-N-methylbenzamide was synthesized by the production method F described in Production Example 6.

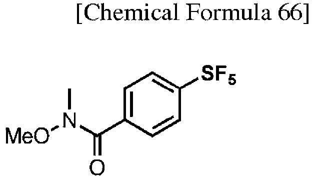

More specifically, it was synthesized from 4-[(trityl)thio]-N-methoxy-N-methylbenzamide using NEt₄Cl in MeCN in accordance with the production method F. 4-(pentafluorosulfanyl)-N-methoxy-N-methylbenzamide (17.8 mg, yield: 61%) was obtained as a white powder through purification by silica column chromatography using (hexane/diethyl ether = 1:1 (volume ratio)) as an eluent.

¹H NMR (400 MHz, CDCl₃) δ 7.83 - 7.75 (m, 4H), 3.55 (s, 3H), 3.38 (s, 3H).

¹³C NMR (100 MHz, CDCl₃) δ 167.8, 155.0 (quint, ²J_{CF} = 17.6 Hz), 137.4, 128.7, 125.8 (quint, ³J_{CF} = 4.6 Hz), 61.3, 33.2.

¹⁹F NMR (375 MHz, CDCl₃) δ 83.5 (quint, ²J_{FF, ax} = 149.3 Hz), 62.6 (d, ²J_{FF, eq} = 149.6 Hz).

### [Example 50]

4-(pentafluorosulfanyl)benzophenone was synthesized by the production method F described in Production Example 6.

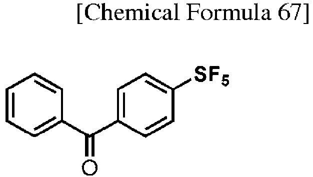

More specifically, it was synthesized from 4-[(trityl)thio]benzophenone using NEt₄Cl in MeCN in accordance with the production method F. 4-(pentafluorosulfanyl)benzophenone (22.3 mg, yield: 72%) was obtained as a white powder through purification by silica column chromatography using (hexane/methylene chloride = 10:1 (volume ratio)) as an eluent.

¹H NMR (400 MHz, CDCl₃) δ 6.68 - 6.58 (m, 1H), 6.55 (dd, J = 8.4, 1.3 Hz, 0H), 6.42 - 6.36 (m, 0H), 6.30 - 6.19 (m, 1H).

¹⁹F NMR (375 MHz, CDCl₃) δ 83.1 (quint, 2JFF, ax = 151.3 Hz), 62.6 (d, 2JFF, eq = 150.2 Hz).

### [Example 51]

5-Methoxy-1-[4-(pentafluorosulfanyl)phenyl]pentan-1-one was synthesized.

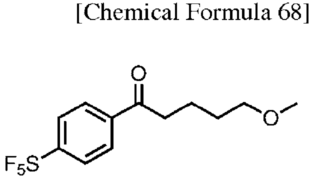

More specifically, THF (3.6 mL) was added to a 15 mL vial containing 4-(pentafluorosulfanyl)-N-methyl-N-methoxybenzamide (0.34 mmol, 100 mg) under nitrogen, and a Grignard reagent solution (0.29 M, 3.6 mL) was further added with stirring at 0°C. Subsequently, this solution was removed from an ice bath and stirred at room temperature for 2 hours. Thereafter, 6 M hydrochloric acid (3.0 mL) was added to the solution at 0°C, and the resulting mixture was stirred for 10 minutes. The obtained reaction solution was neutralized with saturated sodium bicarbonate and extracted with DCM. All organic layers were then combined and dried over anhydrous sodium sulfate, and the solvent was removed using a rotary evaporator to obtain 5-methoxy-1-[4-(pentafluorosulfanyl)phenyl]pentan-1-one (108.2 mg, yield: 99%). It should be noted that this compound was used in the next synthesis without further purification.

¹H NMR (400 MHz, CDCl₃) δ 8.04 (d, J = 8.5 Hz, 2H), 7.86 (d, J = 8.5 Hz, 2H), 3.43 (t, J = 6.3 Hz, 2H), 3.33 (s, 3H), 3.03 (t, J = 7.2 Hz, 2H), 1.84 (m, 2H), 1.67 (m, 2H).

¹⁹F NMR (376 MHz, CDCl₃) δ 83.0 (quint, ²J_{FF, ax} = 150.2 Hz, 1F), 62.5 (d, ²J_{FF, eq} = 149.0 Hz, 4F).

¹³C NMR (101 MHz, CDCl₃) δ 198.7, 139.2, 132.4 (quint, ²J_{CF} = 61.9 Hz), 128.6, 126.6 (quint, ³J_{CF} = 4.7 Hz), 72.6, 58.8, 38.8, 29.2, 21.0.

The Grignard reagent solution was prepared as follows.

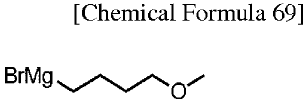

Anhydrous THF (30 mL) and 1-bromo-4-methoxybutane (3 g, 18 mmol) were added to a 100 mL two-necked round bottom flask equipped with a 0°C cooler and containing magnesium cuttings (0.47 g, 1.1 equivalents) and iodine (20 mg) at room temperature with stirring. This reaction mixture was heated to 50°C in an oil bath and stirred overnight. After the reaction, the obtained Grignard reagent solution (0.29M from a titration test) was used without further purification for synthesizing 5-methoxy-1-[4-(pentafluorosulfanyl)phenyl]pentan-1-one.

### [Example 52]

N-{ 5-methoxy-1-[4-(pentafluorosulfanyl)phenyl]pentylidene }hydroxylamine was synthesized.

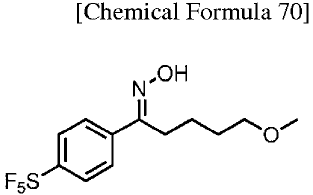

More specifically, hydroxylamine hydrochloride (47.3 mg, 0.68 mmol, 2.0 equivalents) and sodium acetate (55.8 mg, 0.68 mmol, 2.0 equivalents) were added to 5-methoxy-1-[4-(pentafluorosulfanyl)phenyl]pentan-1-one (108.2 mg, 0.34 mmol) dissolved in ethanol (1.7 mL), and the resulting mixture was stirred at room temperature. After 3 hours, this solution was quenched with water and extracted with DCM. All organic layers were combined and dried over anhydrous sodium sulfate, and the solvent was removed using a rotary evaporator to obtain N-{5-methoxy-1-[4-(pentafluorosulfanyl)phenyl]pentylidene } hydroxylamine (101.1 mg, yield: 89%). It should be noted that this compound was used in the next synthesis without further purification.

¹H NMR (400 MHz, CDCl₃) δ 7.75 (d, J = 9.0 Hz, 2H), 7.68 (d, J = 8.7 Hz, 2H), 3.41 (t, J = 6.0 Hz, 2H), 3.33 (s, 3H), 2.84 (t, J = 7.4 Hz, 2H), 1.65 (m, 4H).

¹⁹F NMR (376 MHz, CDCl₃) δ 84.2 (quint, 2J_{FF, ax} = 150.0 Hz, 1F), 62.8 (d, 2J_{FF, eq} = 150.0 Hz, 4F).

¹³C NMR (101 MHz, CDCl₃) δ 158.1, 154.2 (quint, ²J_{CF} = 17.3 Hz), 139.0, 126.6, 126.4 (quint, ³J_{CF} = 4.6 Hz), 72.4, 58.8, 29.6, 25.7, 23.0.

### [Example 53]

Pentafluorosulfanyl-fluvoxamine was synthesized.

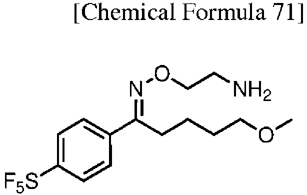

More specifically, in a 15 mL glass vial in an argon-filled glove box, sodium hydride (5.8 mg, 0.24 mmol, 2.0 equivalents) was added to N-{5-methoxy-1-[4-(pentafluorosulfanyl)phenyl]pentylidene }hydroxylamine (40.1 mg, 0.12 mmol) dissolved in DMF (0.7 mL), and the resulting mixture was stirred at room temperature for 3 hours. Thereafter, 2-chloroethylamine hydrochloride (14.0 mg, 0.12 mmol, 1.0 equivalents) was added to this reaction mixture, and the resulting mixture was removed from the glove box and stirred at room temperature for 12 hours. The obtained reaction mixture was quenched with water and extracted with DCM. All organic layers were combined and dried over anhydrous sodium sulfate, the solvent was removed using a rotary evaporator, and the crude material was purified by silica column chromatography (DCM/methanol = 10:1) to obtain pentafluorosulfanyl-fluvoxamine (12.0 mg, yield: 32%) as a colorless oil.

¹H NMR (400 MHz, CDCl₃) δ 7.75 - 7.70 (m, 4H), 4.24 (t, J = 4.7 Hz, 2H), 3.38 (t, J = 5.8 Hz, 2H), 3.32 (br, 2H), 3.04 (s, 1H), 2.79 (t, J = 6.6 Hz, 2H), 1.67 - 1.58 (m, 4H).

¹⁹F NMR (376 MHz, CDCl₃) δ 84.3 (quint, ²J_{FF, ax} = 151.3 Hz, 1F), 62.8 (d, ²J_{FF, eq} = 151.0 Hz, 4F).

¹³C NMR (101 MHz, CDCl₃) δ 157.1, 154.1 (quint, ²J_{CF} = 17.5 Hz), 139.0, 126.6, 126.3 (quint, ³J_{CF} = 4.7 Hz), 76.6, 72.3, 58.8, 41.6, 29.7, 26.1, 23.2.

### [Example 54]

2-Methyl-5-[(trityl)thio]pyrimidine was synthesized using a Pd[cinnamyl](L)OTf catalyst and a Pd[allyl](L)OTf catalyst, which are composed of palladium complexes in which various phosphine ligands (L) are coordinated. The compounds listed in Table 1 were used as phosphine ligands.

### (1) Synthesis of Pd[cinnamyl](L)OTf catalyst and Pd[allyl](L)OTf catalyst

In a 15 mL glass vial in a glove box under an argon atmosphere, silver trifluoromethanesulfonate (AgOTf, 0.1 mmol, 2.0 equivalents) was added to a stirred mixture of THF (1.0 mL) and allylpalladium chloride dimer or Pd[cinnamyl]Cl dimer (0.05 mmol, 1.0 equivalent). The resulting mixture in which silver chloride was formed as a white precipitate was stirred at room temperature for 1 hour. The white precipitate in the reaction mixture was removed with a syringe filter, and the filtrate was added to a 50 mL glass vial containing the phosphine ligand (0.1 mmol, 2.0 equivalents). After stirring at room temperature for 2 hours, hexane (10 mL) was added to the reaction solution to obtain a powdery or crystalline precipitate. The supernatant was removed by decantation, and the residue was washed three times with hexane (5 mL). The residue was evaporated under reduced pressure to obtain the desired Pd[cinnamyl](L)OTf catalyst or Pd[allyl](L)OTf catalyst as a powder or crystal.

### (2) Synthesis of 2-methyl-5-[(trityl)thio]pyrimidine

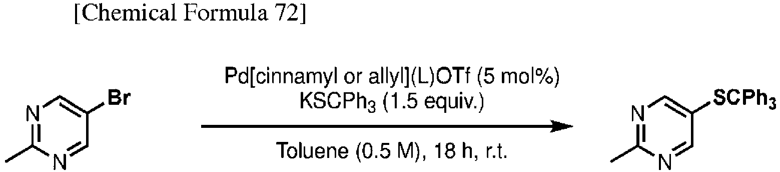

A test tube dried in an oven was moved into a glove box under an argon atmosphere. 5-Bromo-2-methylpyrimidine (0.2 mmol) was added to a mixture of Pd[cinnamyl](L)OTf catalyst or Pd[allyl](L)OTf catalyst (5 mol%) and toluene (0.5 M) in this test tube, and the test tube was then cooled in a refrigerator at -30°C for 0.5 hours. KSTr (KSCPh₃) (94.3 mg, 0.3 mmol, 1.5 equivalents) was added to this mixture with vigorous stirring at room temperature. After sealing with an aluminum cap with a septum using a vial crimper, the test tube was taken out of the glove box, and the resulting mixture was stirred at room temperature for 18 hours. The reaction mixture was diluted with CDCl₃, and the NMR yield of 2-methyl-5-[(trityl)thio]pyrimidine was determined by ¹H NMR.

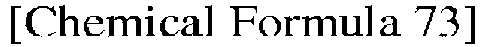

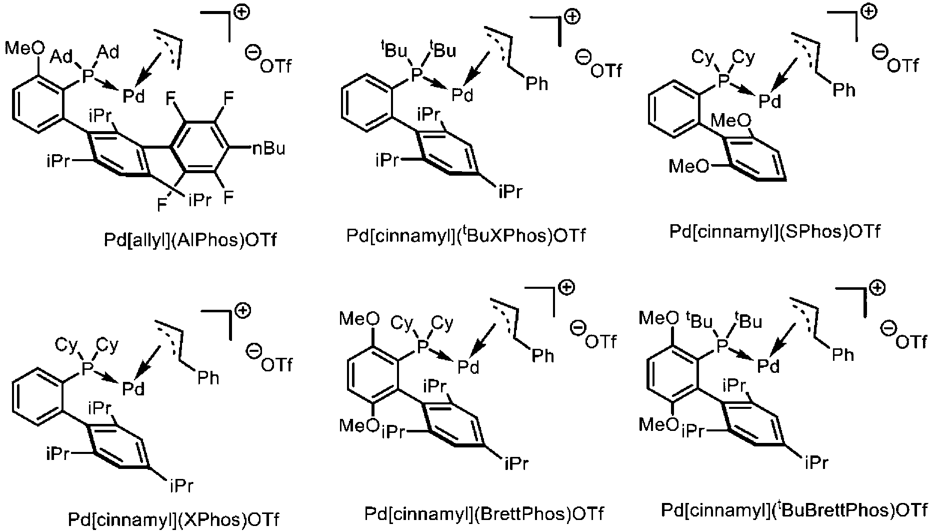

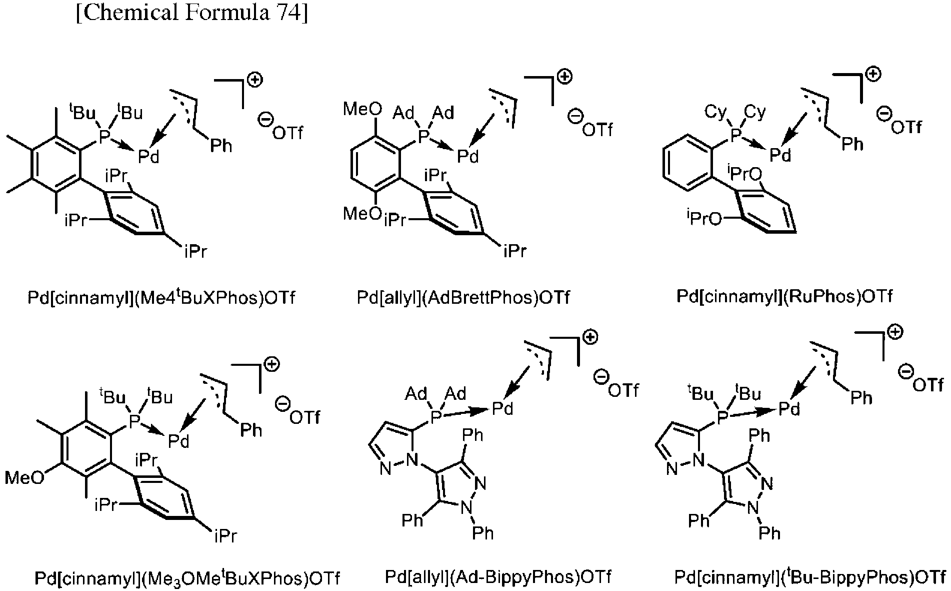

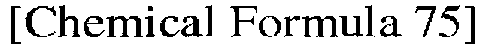

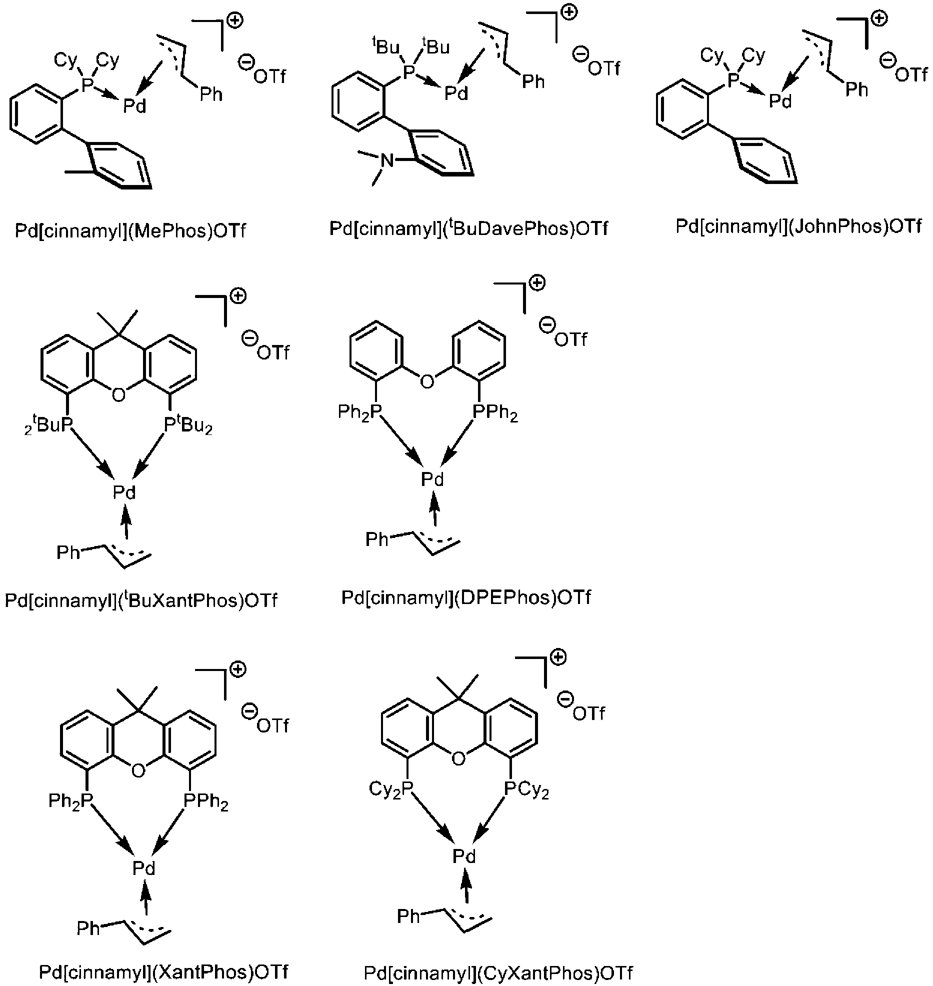

**[Table 1]**

| Catalyst | Phosphine ligand | Yield (%) |
|---|---|---|
| 4-1 | AlPhos | 99 |
| 4-2 | ^{t}BuXPhos | 15 |
| 4-3 | Sphos | <1 |
| 4-4 | Xphos | <1 |
| 4-5 | BrettPhos | 19 |
| 4-6 | ^{t}Bu-BrettPhos | 15 |
| 4-7 | Me₄^{t}BuXPhos | 11 |
| 4-8 | Ad-BrettPhos | 25 |
| 4-9 | RuPhos | <1 |
| 4-10 | Me₃(OMe)^{t}BuXPhos | 9 |
| 4-11 | Ad-BippyPhos | 20 |
| 4-12 | ^{t}Bu-BippyPhos | 50 |
| 4-13 | MePhos | <1 |
| 4-14 | ^{t}BuDavePhos | <1 |
| 4-15 | XantPhos | 33 |
| 4-16 | ^{t}BuXantPhos | <1 |
| 4-17 | DPEPhos | 11 |
| 4-18 | CyXantPhos | 48 |
| 4-19 | JohnPhos | <1 |

The NMR yield of 2-methyl-5-[(trityl)thio]pyrimidine synthesized using each Pd[cinnarnyl](L)OTf catalyst is shown in Table 1. The synthesis of 2-methyl-5-[(trityl)thio]pyrimidine was confirmed in all cases no matter which catalyst was used, but the Pd[cinnamyl](L)OTf catalyst using ^{t}BuXPhos, AlPhos, ^{t}Bu-BippyPhos, CyXantPhos, XantPhos, Ad-BrettPhos, Ad-BippyPhos, BrettPhos, or ^{t}Bu-BrettPhos as a phosphine ligand resulted in a favorable yield.

### [Example 55]

Methyl 4-[(trityl)thio]benzoate was synthesized by the production method E described in Production Example 5.

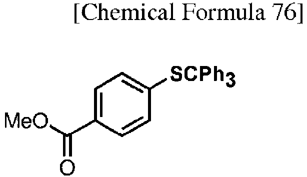

More specifically, it was prepared from methyl 4-iodobenzoate (0.2 mmol, 52.4 mg) and the catalyst 3 (5 mol%, 9.1 mg) in accordance with the production method E. After the reaction, purification was carried out by silica column chromatography (hexane/ethyl acetate = 10:1 (volume ratio)) to obtain methyl 4-[(trityl)thio]benzoate (74.7 mg, yield: 91%) as a white powder.

¹H NMR (400 MHz, CDCl₃) δ 7.63 (d, J = 8.3 Hz, 2H), 7.44 - 7.34 (m, 6H), 7.29 - 7.13 (m, 9H), 7.02 - 6.94 (m, 2H), 3.81 (s, 3H).

¹³C NMR (101 MHz, CDCl₃) δ 166.9, 144.1, 142.5, 131.4, 130.1, 129.2, 128.0, 127.2, 71.1, 52.2.

### [Example 56]

2-Benzyloxy-5-[(trityl)thio]pyrimidine was synthesized by the production method E described in Production Example 5.

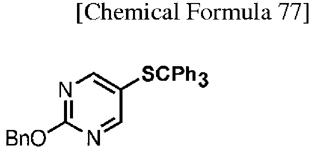

More specifically, it was prepared from 2-benzyloxy-5-iodopyrimidine (0.2 mmol, 62.4 mg) and the catalyst 3 (5 mol%, 9.1 mg) in accordance with the production method E. After the reaction, purification was carried out by silica column chromatography (hexane/ethyl acetate = 10:1 (volume ratio)) to obtain 2-benzyloxy-5-[(trityl)thio]pyrimidine (83.8 mg, yield: 91%) as a white powder.

¹H NMR (400 MHz, CDCl₃) δ 7.92 (s, 2H), 7.46 - 7.37 (m, 8H), 7.36 - 7.08 (m, 12H), 5.32 (s, 2H).

¹³C NMR (101 MHz, CDCl₃) δ 165.4, 164.5, 143.8, 136.2, 129.8, 128.5, 128.1, 128.0, 127.2, 121.6, 71.7, 69.4.

### [Example 57]

2,4-dimethoxy-5-[(trityl)thio]pyrimidine was synthesized by the production method E described in Production Example 5.

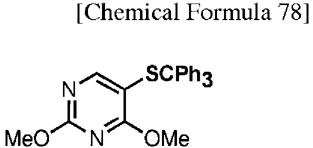

More specifically, it was prepared from 2,4-dimethoxy-5-iodopyrimidine (0.2 mmol, 53.2 mg) and the catalyst 3 (5 mol%, 9.1 mg) in accordance with the production method E. After the reaction, purification was carried out by silica column chromatography (hexane/ethyl acetate = 10:1 (volume ratio)) to obtain 2,4-dimethoxy-5-[(trityl)thio]pyrimidine (72.1 mg, yield: 87%) as a white powder.

¹H NMR (400 MHz, CDCl₃) δ 7.76 (s, 1H), 7.50 - 7.39 (m, 6H), 7.27 - 7.12 (m, 9H), 3.88 (s, 3H), 3.63 (s, 3H).

¹³C NMR (101 MHz, CDCl₃) δ 171.5, 165.3, 144.2, 130.0, 127.7, 126.9, 107.8, 71.7, 55.1, 54.1.

### [Example 58]

2-Fluoro-5-[(trityl)thio]pyridine was synthesized by the production method E described in Production Example 5.

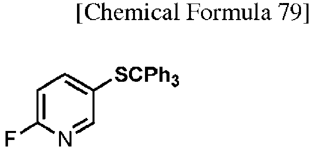

More specifically, it was prepared from 2-fluoro-5-iodopyridine (0.2 mmol, 44.6 mg) and the catalyst 3 (5 mol%, 9.1 mg) in accordance with the production method E. After the reaction, purification was carried out by silica column chromatography (hexane/ethyl acetate = 20:1 (volume ratio)) to obtain 2-fluoro-5-[(trityl)thio]pyridine (64.6 mg, yield: 87%) as a white powder.

¹H NMR (400 MHz, CDCl₃) δ 7.95 - 7.69 (m, 1H), 7.46 - 7.37 (m, 6H), 7.31 - 7.14 (m, 10H), 6.54 (ddd, J = 8.5, 3.0, 0.6 Hz, 1H).

¹³C NMR (101 MHz, CDCl₃) δ 163.5 (d, J = 241.8 Hz), 153.9 (d, J = 14.9 Hz), 147.9 (d, J = 8.6 Hz), 144.0, 129.9, 128.1, 127.1, 109.1 (d, J = 37.7 Hz), 71.7.

¹⁹F NMR (376 MHz, CDCl₃) δ -67.9 (d, J = 6.7 Hz).

### [Example 59]

2-Fluoro-4-[(trityl)thio]pyridine was synthesized by the production method E described in Production Example 5.

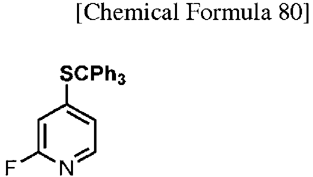

More specifically, it was prepared from 2-fluoro-4-iodopyridine (0.2 mmol, 44.6 mg) and the catalyst 3 (5 mol%, 9.1 mg) in accordance with the production method E. After the reaction, purification was carried out by silica column chromatography (hexane/ethyl acetate = 20:1 (volume ratio)) to obtain 2-fluoro-4-[(trityl)thio]pyridine (54.9 mg, yield: 74%) as a white powder.

¹H NMR (400 MHz, CDCl₃) δ 7.76 (d, J = 5.6 Hz, 1H), 7.35 (dd, J = 8.1, 1.6 Hz, 6H), 7.32 - 7.21 (m, 9H), 6.71 (dt, J = 5.5, 1.5 Hz, 1H), 6.34 (t, J = 1.4 Hz, 1H).

¹³C NMR (101 MHz, CDCl₃) δ 163.22 (d, J = 238.6 Hz), 154.21 (d, J = 8.7 Hz), 146.16 (d, J = 16.0 Hz), 143.1, 130.0, 128.3, 127.7, 120.85 (d, J = 3.8 Hz), 108.54 (d, J = 40.4 Hz), 71.1.

¹⁹F NMR (376 MHz, CDCl₃) δ 68.5.

### [Example 60]

N-(3-chloro-4-(3-fluorobenzyloxy)phenyl)-6-[(trityl)thio]quinazolin-4-amine was synthesized by the production method E described in Production Example 5.

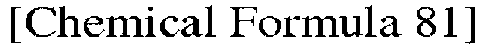

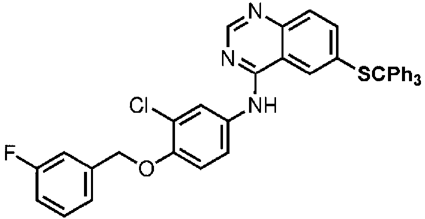

More specifically, it was prepared from N-(3-chloro-4-(3-fluorobenzyloxy)phenyl)-6-iodoquinazolin-4-amine (0.2 mmol, 101.1 mg) and the catalyst 3 (10 mol%, 18.2 mg) in accordance with the production method E. After the reaction, purification was carried out by silica column chromatography (hexane/ethyl acetate = 2:1 (volume ratio)) to obtain N-(3-chloro-4-(3-fluorobenzyloxy)phenyl)-6-[(trityl)thio]quinazolin-4-amine (87.7 mg, yield: 67%) as a white powder.

¹H NMR (400 MHz, CDCl₃) δ 8.64 (s, 1H), 7.75 (d, J = 2.6 Hz, 1H), 7.72 - 7.59 (m, 2H), 7.50 - 7.40 (m, 6H), 7.41 - 7.33 (m, 3H), 7.33 - 7.22 (m, 9H), 7.03 (td, J = 8.1, 1.6 Hz, 1H), 6.98 (d, J = 8.9 Hz, 1H), 6.89 (d, J = 1.5 Hz, 1H), 6.55 (s, 1H), 5.17 (s, 2H).

### [Example 61]

5- { 2-ethoxy-5-[(trityl)thio]-phenyl }-1-methyl-3-propyl-6H-pyrazolo[4, 3-d]pyrimidin-7-one was synthesized by the production method E described in Production Example 5.

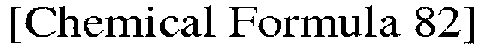

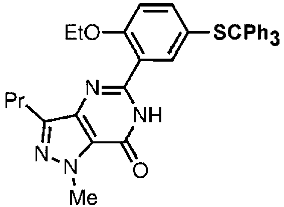

More specifically, it was prepared from (5-(2-ethoxy-5-iodo-phenyl)-1-methyl-3-propyl-6H-pyrazolo[4,3-d]pyrimidin-7-one (0.2 mmol, 87.7 mg) and the catalyst 3 (5 mol%, 9.1 mg) in accordance with the production method E. After the reaction, purification was carried out by silica column chromatography (hexane/ethyl acetate = 2:1 (volume ratio)) to obtain 5-{2-ethoxy-5-[(trityl)thio]-phenyl}-1-methyl-3-propyl-6H-pyrazolo[4,3-d]pyrimidin-7-one (99.7 mg, yield: 85%) as a white powder.

¹H NMR (400 MHz, CDCl₃) δ 10.88 (br, NH), 8.16 (d, J = 2.4 Hz, 1H), 7.49 - 7.41 (m, 6H), 7.28 - 7.11 (m, 9H), 6.94 (dd, J = 8.7, 2.4 Hz, 1H), 6.64 (d, J = 8.7 Hz, 1H), 4.25 (s, 3H), 4.22 - 4.04 (m, 2H), 2.95 - 2.85 (m, 2H), 1.84 (h, J = 7.4 Hz, 2H), 1.53 (t, J = 7.0 Hz, 3H), 1.04 (t, J = 7.4 Hz, 3H).

### [Example 62]

4-[(trityl)thio]-N,N-dipropylbenzenesulfonamide was synthesized by the production method E described in Production Example 5.

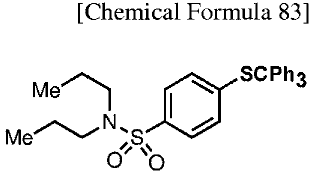

More specifically, it was prepared from 4-iodo-N,N-dipropylbenzenesulfonamide (0.2 mmol, 73.46 mg) and the catalyst 3 (5 mol%, 9.1 mg) in accordance with the production method E. After the reaction, purification was carried out by silica column chromatography (hexane/ethyl acetate = 20:1 (volume ratio)) to obtain -[(trityl)thio]-N,N-dipropylbenzenesulfonamide (98.0 mg, yield: 95%) as a white powder.

¹H NMR (400 MHz, CDCl₃) δ 7.42 - 7.35 (m, 8H), 7.29 - 7.16 (m, 9H), 7.03 (d, J = 8.3 Hz, 2H), 3.01 - 2.92 (m, 4H), 1.47 (h, J = 7.5 Hz, 4H), 0.83 (t, J = 7.4 Hz, 6H).

¹³C NMR (101 MHz, CDCl₃) δ 143.9, 141.6, 138.0, 132.2, 130.0, 128.0, 128.0, 127.2, 126.6, 71.4, 50.0, 22.0, 11.3.

### INDUSTRIAL APPLICABILITY

The present invention provides a production method that allows the synthesis of compounds obtained by introducing a tritylsulfanyl group has into various aryl compounds under relatively mild conditions. In addition, the tritylsulfanyl group-containing aryl compound produced by the method for producing a tritylsulfanyl group-containing aryl compound according to the present invention can be easily converted into an SF₅ group-containing aryl compound by an oxidative fluorination reaction, and is suitable as a raw material substrate for an SF₅ group-containing aryl compound. For this reason, the present invention is useful for introducing an SF₅ group into the medicinal ingredients of medicines and agricultural chemicals, organic materials, and the like.

## Claims

1. A method for producing a tritylsulfanyl group-containing aryl compound,
the method comprising
thiotritylation of a halogenated aryl compound represented by the following general formula (1) using [(triphenylmethyl)sulfanyl]potassium or [(triphenylmethyl)sulfanyl] sodium,
[Chemical Formula 1]
A¹-X (1)
[wherein A¹ is an aryl group which may have a substituent or a heteroaryl group which may have a substituent; and X is a halogen atom]
to produce a tritylsulfanyl group-containing aryl compound represented by the following general formula (2): 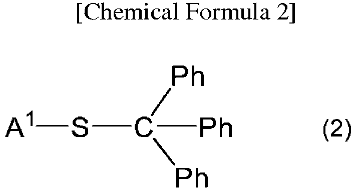
[wherein A¹ is the same as A¹ in the general formula (1), and Ph is a phenyl group].

2. The method for producing a tritylsulfanyl group-containing aryl compound according to Claim 1, further comprising
thiotritylation of the halogenated aryl compound represented by said general formula (1) using a palladium catalyst.

3. The method for producing a tritylsulfanyl group-containing aryl compound according to Claim 2, wherein said palladium catalyst is Pd[cinnamyl](^{t}BuXPhos)OTf or Pd[allyl](AlPhos)OTf.

4. The method for producing a tritylsulfanyl group-containing aryl compound according to Claim 1,
wherein said A¹ is
an aryl group which may have one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a cyano group, a fluorinated formyl group, an amino group, a nitro group, a pyrrolidinyl group, a piperidinyl group, a piperazinyl group, a pyrazolidinyl group, an imidazolidinyl group, a tetrahydrofuranyl group, a 1,3-dioxolanyl group, a tetrahydrothiophenyl group, a 1,2-oxathiolanyl group, a morpholinyl group, and a tetrahydropyranyl group, or
a heteroaryl group which may have one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a cyano group, a fluorinated formyl group, an amino group, a nitro group, a pyrrolidinyl group, a piperidinyl group, a piperazinyl group, a pyrazolidinyl group, an imidazolidinyl group, a tetrahydrofuranyl group, a 1,3-dioxolanyl group, a tetrahydrothiophenyl group, a 1,2-oxathiolanyl group, a morpholinyl group, and a tetrahydropyranyl group.

5. The method for producing a tritylsulfanyl group-containing aryl compound according to Claim 1,
wherein a reaction of said thiotritylation is carried out at 0 to 80°C.

6. A method for producing a pentafluorosulfanyl group-containing aryl compound,
the method comprising:
producing a tritylsulfanyl group-containing aryl compound represented by said general formula (2) from a halogenated aryl compound represented by said general formula (1) by the method for producing a tritylsulfanyl group-containing aryl compound according to any one of Claims 1 to 5; and
synthesizing a pentafluorosulfanyl group-containing aryl compound represented by the following general formula (3) from said tritylsulfanyl group-containing aryl compound by an oxidative fluorination reaction using a divalent or higher metal fluoride and an organic salt containing a quaternary ammonium cation or a quaternary phosphonium cation
[Chemical Formula 3]
A¹⁻SF₃ (3)
[wherein A¹ is the same as A¹ in the general formula (1)].

7. The method for producing a pentafluorosulfanyl group-containing aryl compound according to Claim 6,
wherein said oxidative fluorination reaction is carried out at -40 to 130°C.
